# EUROPEAN PATENT APPLICATION

(11) **EP 1 179 540 A1**
(43) Date of publication of application: **13.02.2002**
(21) Application number: 00929805.0
(22) Date of filing: 19.05.2000
(51) Int. Cl.: C07K 14/47, C12P 21/02, C07K 16/18, C12P 21/08, G01N 33/50, G01N 33/15, G01N 33/563, A61K 38/16, A61K 39/395

(54) **NOVEL POLYPEPTIDE**

(30) Priority: 20.05.1999 JP 14022999
(71) Applicant: Takeda Chemical Industries, Ltd., Osaka-shi, Osaka 541-0045 (JP)
(72) Inventor: Itoh, Yasuaki, Tsuchiura-shi, Ibaraki 300-0832 (JP); Mogi, Shinichi, Kitasoma-gun, Ibaraki 302-0121 (JP); Tanaka, Hideyuki, Tsukuba-shi, Ibaraki 305-0821 (JP); Ohkubo, Shoichi, Usiku-shi, Ibaraki 300-1234 (JP); Ogi, Kazuhiro, Tsukuba-shi, Ibaraki 305-0045 (JP)
(74) Representative: Keller, Günter, Dr.
(86) International application number: JP0003221
(87) International publication number: WO0071581

(57) **Abstract**

The present invention relates to a novel secretory protein or salts thereof, methods of manufacturing the protein, a pharmaceutical composition comprising the protein, an antibody to the protein, screening methods/screening kits for a compound or salts thereof that promotes or inhibits activities of the protein, etc.

The protein of the present invention can be used as prophylactic/therapeutic agents for various diseases such as cancer, immunologicl diseases, pulmonary dysfunction, liver dysfunction, infection or gastrointestinal disorders, etc. The antibody of the present invention can be used for quantification of the protein of the present invention in the test solution. Furthermore, the protein of the present invention is useful as the reagent for screening a compound that either promotes or inhibits activities of the protein of the present invention.

## Description

### FIELD OF THE INVENTION

The present invention relates to a novel secretory polypeptide, new uses of the secretory polypeptide.

### BACKGROUND ART

Living bodies exert harmonized regulations involved in genesis, differentiation, growth, repairment and maintenance of homeostasis, via intercellular or intertissular transmission of information; in most of these occasions, extracellularly secreted protein factors play an important role as the information transmitters. For example, a series of secretory factors (humoral factor) called as cytokines occur as polypeptides modified by sugar chains, with their molecular weights ranging from 20,000 to 50,000, and they are characterized as hormone-like molecules mainly exhibiting effects on immune and hematopoietic systems. Various monokines found as macrophage activating factors to be released from monocytes and migration-inhibitory factors, interferon α,β and γ characterized by activating relevant cells to induce anti-virus activities, as well as polypeptides known as various lymphokines which are produced at T cells and postulated as lymphocyte proliferative differentiation factors have been consecutively reported so far, whereby they are usually classified as a member of cytokines in general. One of the characteristics of cytokines is known to predominantly exert their activities by either autocrine or paracrine modes. On the other hand, humoral factors including peptide hormones and proliferative factors which are derived from endocrine tissues exhibit activities by endocrine properties; therefore, investigations searching for possible correlations between most of these humoral factors and diseases as well as research and development to pursue feasibility of them as ethical drugs are currently performed extensively.

In the meantime, most of these protein factors indicative of being important for living bodies have been mostly discovered either by using their intrinsic biological activities as the index or by virtue of structural similarity to those of previously known physiologically active polypeptides. However, given the sophisticated maintenance of homeostasis observed in higher animals including mammalians, it is well postulated that lots of unknown factors except for the physiologically active polypeptides or peptides, which is publicly known are present and contribute to important functions. In recent years, by large scale determinations of base sequences in cDNA library not only from humans but also various organisms, and the genome structure analysis project, quite a few new candidates of genes have been verified; accordingly, computerized information processing technology is widely employed to anticipate possible functions of these gene products, prompting researchers to apply these findings to biology, medicine, veterinary medicine and agronomy [Trends in Biotechnology, Vol. 14, 294 (1996)]. Nevertheless, most of these sequence information is generally fragmental and incorrect, thereby leading to difficulty in selecting brand-new useful genes directly from them under the current situation. For example, partial miscoding is occasionally observed among the sequence information obtained by a large scale base sequence determination; therefore, it is necessary for us to rely on correct information on the base sequences and to objectively judge whether secretory factors (humoral factors) could be obtained by these base sequences so that we obtain correct evidence about open reading frame (ORF) and amino acid sequences. The present invention successfully verifies a novel humoral factor through a large scale cDNA sequence analysis and subsequent detailed analysis of relevant information from the viewpoint as stated above.

The objective of this invention is to disclose a novel humoral factor, thereby providing novel polypeptides, its partial peptides or salts thereof, recombinant vectors, transformants, which are applicable to biology, medicine, pharmaceutical field and veterinary medicine, manufacturing methods of the polypeptides, pharmaceutical components containing the polypeptides and partial polypeptides, as well as antibodies directed against the polypeptides.

### DISCLOSURE OF THE INVENTION

As a result of extensive investigations to solve the above-stated issues, the present inventors have selected cDNAs encoding novel secretory polypeptides among a large scale cDNA base sequence data base, followed by identification of plural numbers of structures of novel secretory polypeptides which meet the objectives of this investigation. Based on these findings, the present inventors have continued further extensive studies and as a result, have come to accomplish the present invention.

Thus, the present invention relates to the following features.
(1) A polypeptide comprising the same or substantially the same amino acid sequence as at least one (preferably, one) amino acid sequence selected among either SEQ ID NO: 1 or SEQ ID NO:15, or a salt thereof.
(2) A manufacturing method for the polypeptide or its salt according to (1), which comprises culturing the transformant previously transformed with the recombinant vector containing the DNA encoding the polypeptide and accumulating the polypeptide according to (1).
(3) An antibody to the polypeptide or its salt according to (1).
(4) A method of screening a compound or its salt that either promotes or inhibits the activity of the polypeptide or its salt according to (1), which comprises using the polypeptide or its salts according to (1).
(5) A kit for screening a compound or its salt that either promotes or inhibits the activity of the polypeptide or its salt according to (1), comprising the polypeptide or its salt according to (1).
(6) A compound or its salt that either promotes or inhibits the activity of the polypeptide or its salt according to (1), which is obtainable using the screening method according to (4) or the screening kit according to (5).
(7) A pharmaceutical composition comprising a compound or its salt that either promotes or inhibits the activity of the polypeptide or its salt according to (1), which is obtainable using the screening method according to (4) or the screening kit according to (5).
(8) A pharmaceutical comprising the polypeptide or its salt according to (1).
   The present invention further relates to the following features.
(9) A polypeptide according to (1), wherein substantially the same amino acid sequence as one amino acid sequence selected among amino acid sequences represented by SEQ ID NO: or SEQ ID NO:15 possesses not less than about 50% homology (preferably not less than about 60% homology, and further preferably not less than about 70% homology, more preferably not less than about 80% homology, much more preferably not less than about 90% homology, and most preferably approximately 95% homology) to one amino acid sequence selected among the amino acid sequences represented by either SEQ ID NO:1 or SEQ ID NO:15.
(10) A polypeptide according to (1), wherein the polypeptide contains ① substantially the same amino acid sequence as at least one amino acid sequence selected among amino acid sequences represented by SEQ ID NO:1 or SEQ ID NO:15, of which at least 1 or 2 (preferably approximately 1 to 30) amino acids are deleted; ② the amino acid sequence selected among the amino acid sequences shown by SEQ ID NO: 1 or SEQ ID NO: 15, to which at least I or 2 (preferably approximately 1 to 30) amino acids are added; ③ the amino acid sequence selected among the amino acid sequences represented by SEQ ID NO:1 or SEQ DD NO:15, in which at least 1 or 2 (preferably approximately 1 to 30) amino acids are substituted; or ④ the amino acid sequence containing a combination of these amino acid sequences.
(11) DNA encoding the said polypeptide according to (1).
(12) A recombinant vector which contains DNA encoding the said polypeptide according to (1).

Furthermore, the polypeptide of the present invention can be applied to fundamental studies including molecular weight marker, tissue marker, chromosome mapping, identification of genetic diseases, designing of primers and probes.

### BRIEF DESCRIPTION OF THE DRAWINGS

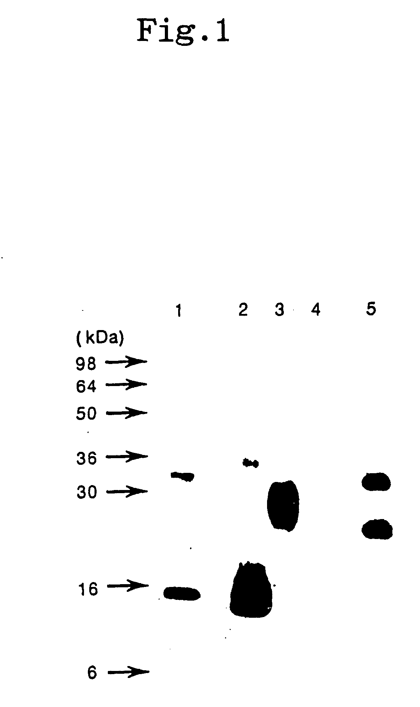

FIG. 1 shows the Western Blot analysis patterns indicating confirmation of secretion of each clone from animal cells, which was performed in Examples 16 - 20. The culture supernatant equivalent to 1/16 well which was recovered from 6-well plate was subjected to development with use of 10-25% SDS-PAGE as the developing solvent, followed by Western Blot analysis with use of anti-FLAG antibody.

In this FIG., lane 1, lane 2, lane 3, lane 4 and lane 5 represent electrophoresis of the culture supernatant of COS7 cells expressing TGC-480, TGC-711, TGC-714, TGC-715 and TGC-623, respectively.

### BEST MODE FOR CARRYING OUT THE INVENTION

The polypeptide (hereinafter sometimes merely referred to as the polypeptide of the present invention) comprising at least one amino acid sequence selected from SEQ ID NO:1 or SEQ ID NO:15 of the present invention may be any polypeptide derived from any cells (e.g., liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophile, basophile, eosinophile, monocyte), megakaryocyte, synovial cells, chondrocytes, bone cells, osteoblasts, osteoclasts, mammary gland cells, interstitial cells, or the corresponding precursor cells, stem cells, or cancer cells, etc.), or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, cartilage, joint and skeletal muscle, etc. from human and other warm-blooded animals (e.g., guinea pigs, rats, mice, chicken, rabbits, swine, sheep, bovine, monkeys, etc.). The polypeptide may also be either a recombinant polypeptide or a synthetic polypeptide.

The polypeptide of the present invention comprises a signal peptide therein, permitting extracellular secretion of the said polypeptide efficiently.

The amino acid sequence which has substantially the same amino acid sequence as one amino acid sequence selected from SEQ ID NO: 1 or SEQ ID NO: 15 includes an amino acid sequence having not less than about 50% homology, preferably not less than about 60% homology, and further preferably not less than about 70% homology, more preferably not less than about 80% homology, much more preferably not less than about 90% homology, and most preferably approximately 95% homology compared with that of one amino acid sequence selected among the amino acid sequences represented by either SEQ ID NO:1 or SEQ ID NO:15.

The polypeptide which has substantially the same amino acid sequence as one amino acid sequence selected from SEQ ID NO:1 or SEQ ID NO:15 of the present invention includes a polypeptide having substantially the same amino acid sequence as that of one amino acid sequence selected from above-stated SEQ ID NO:1 or SEQ ID NO:15 and furthermore, having substantially the equivalent properties as those of the polypeptide having one amino acid sequence selected among SEQ ID NO: 1 or SEQ ID NO: 15.

Examples of the substantially equivalent properties include an exertion of its activity in that the polypeptide is extracellularly secreted and works as a humoral factor, etc. The term "substantially equivalent " is used to mean that the nature of the properties is the same. Therefore, although it is preferred that the properties such as the secretory pattern and the solubility, etc. be equivalent (e.g., about 0.1- to 100-fold, preferably about 0.5- to 10-fold, more preferably 0.5- to 2-fold), quantitative factors such as a level of the properties, a molecular weight of the polypeptide, etc. may differ.

Specifically, examples of the said polypeptide include a polypeptide containing the amino acid sequence comprising the 22^{nd} amino acid to the 125^{th} amino acid represented by SEQ ID NO:1; a polypeptide containing the amino acid sequence comprising the 24^{th} amino acid to the 121^{st} amino acid represented by SEQ ID NO:2; a polypeptide containing the amino acid sequence comprising the 20^{th} amino acid to the 223^{rd} amino acid represented by SEQ ID NO:3; a polypeptide containing the amino acid sequence comprising the 22^{nd} amino acid to the 248^{th} amino acid represented by SEQ ID NO:4; a polypeptide containing the amino acid sequence comprising the 20^{th} amino acid to the 173^{rd} amino acid represented by SEQ ID NO:5; a polypeptide containing the amino acid sequence comprising the 21^{st} amino acid to the 261^{st} amino acid represented by SEQ ID NO:6; a polypeptide containing the amino acid sequence comprising the 31^{st} amino acid to the 243^{rd} amino acid represented by SEQ ID NO:7; a polypeptide containing the amino acid sequence comprising the 19^{th} amino acid to the 149^{th} amino acid represented by SEQ ID NO:8; a polypeptide containing the amino acid sequence comprising the 21^{st} amino acid to the 136^{th} amino acid represented by SEQ ID NO:9; a polypeptide containing the amino acid sequence comprising the 23^{rd} amino acid to the 123^{rd} amino acid represented by SEQ ID NO:10; a polypeptide containing the amino acid sequence comprising the 21^{st} amino acid to the 163^{rd} amino acid represented by SEQ ID NO:11; a polypeptide containing the amino acid sequence comprising the 19^{th} amino acid to the 301^{st} amino acid represented by SEQ ID NO:12; a polypeptide containing the amino acid sequence comprising the 27^{th} amino acid to the 69^{th} amino acid represented by SEQ ID NO:13; a polypeptide containing the amino acid sequence comprising the 24^{th} amino acid to the 69^{th} amino acid represented by SEQ ID NO: 14; and a polypeptide containing the amino acid sequence comprising the 27^{th} amino acid to the 197^{th} amino acid represented by SEQ ID NO: 15.

More specifically, polypeptides containing the following amino acid sequence are included in this invention ;amino acid sequences represented by SEQ ID NO:1 or SEQ ID NO:15, wherein at least 1 or 2 amino acids (preferably approximately 1 to 30 amino acids, more preferably approximately 1 to 10 amino acids, most preferably several (1 to 5) amino acids) are deleted; amino acid sequences represented by SEQ ID NO: 1 or SEQ ID NO:15, wherein at least 1 or 2 amino acids (preferably approximately 1 to 30 amino acids, more preferably approximately 1 to 10 amino acids, most preferably several (1 to 5) amino acids) are added; amino acid sequences represented by SEQ ID NO: 1 or SEQ ID NO: 15, wherein at least 1 or 2 amino acids (preferably approximately 1 to 30 amino acids, more preferably approximately 1 to 10 amino acids, most preferably several (1 to 5) amino acids) are inserted; amino acid sequences represented by SEQ ID NO:1 or SEQ ID NO:15, wherein at least 1 or 2 amino acids (preferably approximately 1 to 30 amino acids, more preferably approximately 1 to 10 amino acids, most preferably several (1 to 5) amino acids) are substituted by other amino acids; or combination of the above-cited amino acid sequences constituting a polypeptide including mutein.

As stated above, in the cases with the amino acid sequence being inserted, deleted or substituted, there is no specific limitation with regard to the position of insertion, deletion and substitution.

Throughout the present specification, the polypeptide is represented in accordance with the conventional way of describing peptides, that is, the N-terminus (amino terminus) at the left hand and the C-terminus (carboxyl terminus) at the right hand. In the polypeptides of the present invention including the polypeptide containing the amino acid sequence shown by SEQ ID NO:1, the C-terminus is usually in the form of a carboxyl group (-COOH) or a carboxylate (-COO⁻) but may be in the form of an amide (-CONH₂) or an ester (-COOR).

Examples of the ester group shown by R include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; and the like. In addition, pivaloyloxymethyl or the like, which is used widely as an ester for oral administration, may also be used.

Where the polypeptide of the present invention contains a carboxyl group (or a carboxylate) at a position other than the C-terminus, its amido derivative or ester derivative of the carboxyl group is also included within the peptide of the present invention. As the ester group in such cases, the ester of the above-stated C-terminus may be used.

Furthermore, examples of the polypeptide of the present invention include variants, wherein the amino group at the N-terminal amino acid residue (e.g., methionine residue) is protected with a protecting group (for example, a C₁₋₆ acyl group such as a C₁₋₆ alkanoyl group, e.g., formyl group, acetyl group, etc.); those wherein the N-terminal region is cleaved in vivo and the glutamyl residue thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins bound to sugar chains.

For the polypeptide or its salts of the present invention, salts with physiologically acceptable acids (e.g., inorganic acids, organic acids) and bases (e.g., alkali metal salts) are used; particularly, physiologically acceptable acid addition salts are preferred. Examples of such salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide or its salts of the present invention may be manufactured by a publicly known purification method from human or other mammalian cells or tissues described above, or by culturing a transformant comprising the DNA encoding the polypeptide of the present invention, as will be later described. Furthermore, the polypeptide or its salts may also be manufactured by the methods for synthesizing peptides, which will also be described hereinafter.

Where the polypeptide or its salts are manufactured from human or mammalian tissues or cells, human or mammalian tissues or cells are homogenized, then extracted with an acid or the like, and the extract can be isolated and purified by a combination of chromatography techniques such as reverse phase chromatography, ion exchange chromatography, and the like.

To synthesize the polypeptide or its salts, or its amides or salts thereof according to the present invention, commercially available resins that are used for peptide synthesis may be used. Examples of such resins include chloromethyl resin, hydroxymethyl resin, benzhydrylamine resin, aminomethyl resin, 4-benzyloxibenzylalcohol resin, 4-methylbenzhydrylamine resin, PAM resin,4-hydroxymethylmethylphenylacetamidomethyl resin, polyacrylamide resin, 4-(2',4'-dimethyoxyphenylhydroxymethyl)phenoxy resin, 4-(2',4'-dimethoxyphenyl-Fmoc-aminoethyl)phenoxy resin, etc. Using these resins, amino acids in which α-amino groups and functional groups on the side chains appropriately are protected are condensed on the resin in the order of the sequence of the objective polypeptide according to various condensation methods publicly known. At the end of the reaction, the polypeptide is liberated from the resin and at the same time, the protecting groups are removed. Then, intramolecular disulfide bond-forming reaction is performed in a highly diluted solution to obtain the objective polypeptide or its amides.

For condensation of the protected amino acids described above, a variety of activation reagents for peptide synthesis can be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides , HOBt esters or HOOBt ester, followed by adding the thus activated protected amino acids to the resin.

Solvents suitable for use to activate the protected amino acids or condense with the resin may be chosen from solvents known to be usable for peptide condensation reactions. Examples of such solvents are acid amides such as N,N-dimethylformamide, N,N-dimethylacetamide, N-methylpyrrolidone, etc. ; halogenated hydrocarbons such as methylene chloride, chloroform, etc.; alcohols such as trifluoroethanol, etc. ; sulfoxides such as dimethylsulfoxide, etc. ; ethers such as pyridine, dioxane, tetrahydrofuran, etc. ; nitriles such as acetonitrile, propionitrile, etc. ; esters such as methyl acetate, ethyl acetate, etc. ; and appropriate mixtures of these solvents. The reaction temperature is appropriately chosen from the range known to be applicable to peptide binding reactions and is usually selected in the range of approximately -20°C to 50°C. The activated amino acid derivatives are used generally in an excess of 1.5 to 4 times. The condensation is examined by a test using the ninhydrin reaction ; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole, whereby influences on the subsequent reactions can be avoided.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, tritylhydrazidation, or the like.

The hydroxyl group of serine can be protected through, for example, its esterification or etherification. Examples of groups appropriately used for the esterification include a lower (C₁₋₆)alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bz1, Cl₂-Bz1, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (ester with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids ; a treatment with a base such as diisopropylethylamine, triethylamine, piperidine or piperazine; and reduction with sodium in liquid ammonia. The elimination of the protecting group by the acid treatment described above is carried out generally at a temperature of approximately - 20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the polypeptide, for example, the α-carboxyl group of the carboxy terminal amino acid is first protected by amidation; the peptide (polypeptide) chain is then extended from the amino group side to a desired length. Thereafter, a polypeptide in which only the protecting group of the N-terminal α-amino group in the peptide chain has been eliminated from the polypeptide and a polypeptide in which only the protecting group of the C-terminal carboxyl group has been eliminated are separately prepared. The two polypeptides are condensed in a mixture of the solvents described above. The details of the condensation reaction are the same as described above. After the protected polypeptide obtained by the condensation is purified, all the protecting groups are eliminated by the method described above to give the desired crude polypeptide. This crude polypeptide is purified by various known purification means. Lyophilization of the major fraction gives the amide of the desired polypeptide.

To prepare the esterified polypeptide, for example, the α-carboxyl group of the carboxy terminal amino acid is condensed with a desired alcohol to prepare the amino acid ester, which is followed by procedure similar to the preparation of the amidated polypeptide above to give the ester form of the desired polypeptide.

The polypeptide or its salts in the present invention can be manufactured by publicly known methods for peptide synthesis. For the methods for peptide synthesis, for example, either solid phase synthesis or liquid phase synthesis may be used. That is, the partial peptide or amino acids that can construct the partial peptide of the present invention are condensed with the remaining part. Where the product contains protecting groups, these protecting groups are removed to give the desired peptide. Publicly known methods for condensation and elimination of the protecting groups are described in 1) - 5) below.
1) M. Bodanszky & M.A. Ondetti : Peptide Synthesis, Interscience Publishers, New York (1966)
2) Schroeder & Luebke : The Peptide, Academic Press, New York (1965)
3) Nobuo Izumiya, et al. : *Peptide Gosei-no-Kiso to Jikken* (Basics and experiments of peptide synthesis), published by Maruzen Co. (1975)
4) Haruaki Yajima & Shunpei Sakakibara : *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the polypeptide of the present invention. When the polypeptide obtained by the above methods is in a free form, the peptide can be converted into an appropriate salt by a publicly known method or by modifications thereof ; when the polypeptide is obtained in a salt form, it can be converted into a free form by a publicly known method or by modifications thereof.

The DNA encoding the polypeptide of the present invention may be any DNAs so long as it contains the base sequence encoding the polypeptide of the present invention described above. Furthermore, the said DNA may be any of genomic DNA, cDNA derived from the cells and tissues described above, and synthetic DNA.

The vector to be used for the library may be any of bacteriophage, plasmid, cosmid and phagemid. The total RNA or mRNA fraction prepared from the cells and tissues described above can be directly amplified by reverse transcriptase polymerase chain reaction (hereinafter abbreviated as RT-PCR).

The DNA encoding the polypeptide of the present invention may be any DNA having at least one base sequence selected from SEQ ID NO: 16 or SEQ ID NO:30 ; or the DNA having the base sequence hybridizable to at least one base sequence selected from SEQ ID NO:16 or SEQ ID NO:30 under highly stringent conditions, and encoding the polypeptide having the properties substantially equivalent to those of the peptide of the present invention (e.g., an immunogenicity), and further, the DNA encoding the polypeptide having substantially the same properties as those of the polypeptide of the present invention.

Specific examples of the said DNA include DNA containing at least one base sequence selected from SEQ ID NO: 16 or SEQ ID NO:30, or the DNA having the base sequence hybridizable to at least one base sequence selected from SEQ ID NO:16 or SEQ ID NO:30 under highly stringent conditions, and encoding the polypeptide having the properties substantially equivalent to those of the peptide of the present invention (e.g., an immunogenicity) and further, the DNA encoding the polypeptide having substantially the same properties as those of the polypeptide of the present invention. More specific examples of the said DNA include the DNA containing at least one base sequence selected from SEQ ID NO: 16 or SEQ ID NO:30, or the DNA having the base sequence hybridizable to at least one base sequence selected from SEQ ID NO:16 or SEQ ID NO:30 under highly stringent conditions, and encoding the polypeptide having the properties substantially equivalent to those of the peptide of the present invention (e.g., a secretory activity) and further, the DNA encoding the polypeptide having substantially the same properties as those of the polypeptide of the present invention.

Specific examples of the DNA hybridizable to at least one base sequence selected from SEQ ID NO:16 or SEQ ID NO:30 under highly stringent conditions include DNA containing approximately not less than 60% homology, preferably approximately not less than 70% homology, more preferably approximately not less than 80% homology, to at least one base sequence selected from SEQ ID NO: 16 or SEQ ID NO:30.

More specifically, examples of the DNA encoding the polypeptide containing the amino acid sequence represented by SEQ ID NO:1 include DNA containing a base sequence represented by SEQ ID NO:16 ; examples of the DNA encoding the polypeptide containing the amino acid sequence represented by SEQ ID NO:2 include DNA containing a base sequence represented by SEQ ID NO:17 ; examples of the DNA encoding the polypeptide containing the amino acid sequence represented by SEQ ID NO:3 include DNA containing a base sequence represented by SEQ ID NO:18 ; examples of the DNA encoding the polypeptide containing the amino acid sequence represented by SEQ ID NO:4 include DNA containing a base sequence represented by SEQ ID NO:19 ; examples of the DNA encoding the polypeptide containing the amino acid sequence represented by SEQ ID NO:5 include DNA containing a base sequence represented by SEQ ID NO:20 ; examples of the DNA encoding the polypeptide containing the amino acid sequence represented by SEQ ID NO:6 include DNA containing a base sequence represented by SEQ ID NO:21 ; examples of the DNA encoding the polypeptide containing the amino acid sequence represented by SEQ ID NO:7 include DNA containing a base sequence represented by SEQ ID NO:22 ; examples of the DNA encoding the polypeptide containing the amino acid sequence represented by SEQ ID NO:8 include DNA containing a base sequence represented by SEQ ID NO:23 ; examples of the DNA encoding the polypeptide containing the amino acid sequence represented by SEQ ID NO:9 include DNA containing a base sequence represented by SEQ ED NO:24 ; examples of the DNA encoding the polypeptide containing the amino acid sequence represented by SEQ ID NO:10 include DNA containing a base sequence represented by SEQ ID NO:25 ; examples of the DNA encoding the polypeptide containing the amino acid sequence represented by SEQ ID NO:11 include DNA containing a base sequence represented by SEQ ID NO:26 ; examples of the DNA encoding the polypeptide containing the amino acid sequence represented by SEQ ID NO:12 include DNA containing a base sequence represented by SEQ ID NO:27 ; examples of the DNA encoding the polypeptide containing the amino acid sequence represented by SEQ ID NO:13 include DNA containing a base sequence represented by SEQ ID NO:28 ; examples of the DNA encoding the polypeptide containing the amino acid sequence represented by SEQ ID NO:14 include DNA containing a base sequence represented by SEQ ID NO:29 ; examples of the DNA encoding the polypeptide containing the amino acid sequence represented by SEQ ID NO:15 include DNA containing a base sequence represented by SEQ ID NO:30.

The hybridization can be carried out by publicly known methods or by modifications of these methods, for example, according to the method described in Molecular Cloning, 2^{nd} (J. Sambrook et al., Cold Spring Harbor Lab. Press. 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C.

For cloning of the DNA that completely encodes the polypeptide of the present invention, the DNA may be either amplified by PCR using synthetic DNA primers containing a part of the base sequence of the polypeptide of the present invention and the template derived from tissues or cells expressing the polypeptide, or the DNA inserted into an appropriate vector can be selected by hybridization with a labeled DNA fragment or synthetic DNA that encodes a part or entire region of the polypeptide of the present invention, against a library derived from tissues and cells that express the polypeptide in question. The hybridization can be carried out, for example, according to the method described in Molecular Cloning, 2^{nd,} (J. Sambrook et al., Cold Spring Harbor Lab. Press. 1989). The hybridization may also be performed using commercially available library in accordance with the protocol described in the attached instructions.

Substitution of the base sequence of the DNA can be conducted by PCR or publicly known methods such as the Gupped duplex method or the Kunkel method or its modification by using a publicly known kit available as Mutan™-G or Mutan™-K (both manufactured by Takara Shuzo Co., Ltd.).

The cloned DNA encoding the polypeptide can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the polypeptide of the present invention can be manufactured, for example, by (a) excising the desired DNA fragment from the DNA encoding the polypeptide of the present invention, and then (b) ligating the DNA fragment downstream a promoter in an appropriate expression vector.

Examples of the vector include plasmid derived from E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, , etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, λP_{L} promoter, lpp promoter, and T7 promoter, etc. In the case of using bacteria of the genus Bacillus as the host, preferred examples of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin promoter and P10 promoter, etc.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in dhfr gene deficient Chinese hamster cells, selection of the objective gene can also be made on thymidine free media.

If necessary and desired, a signal sequence tha matches with a host is added to the N-terminus of the polypeptide of the present invention. Examples of the signal sequence that can be used are Pho A signal sequence, OmpA signal sequence, etc. in the case of using bacteria of the genus Escherichia as the host ; α-amylase signal sequence, subtilisin signal sequence, etc. in the case of using bacteria of the genus Bacillus as the host ; MF α signal sequence, SUC2 signal sequence, etc. in the case of using yeast as the host ; and insulin signal sequence, α-interferon signal sequence, antibody molecule signal sequence, etc. in the case of using animal cells as the host, respectively.

Using the vector containing the DNA encoding the polypeptide of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichai coli K12 DH 1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207- 21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, and cells derived from Estigmena acrea, etc. ; and for the virus BmNPV, Bombyx mori N cells (BmN cells), etc are used. Examples of the Sf cells which can be used are Sf9 cells (ATCC CRL711) and Sf21 cells [both cells are described in Vaughn, J.L. et al., In Vivo, 13, 213 - 217 (1977)].

As the insect, for example, a larva of Bombyx mori can be used [Maeda, et al., Nature, 315, 592 (1985)].

Examples of animal cells include monkey cells COS7, Vero, Chinese hamster cells CHO (hereinafter referred to as CHO cells), dhfr gene deficient Chinese hamster cells CHO (hereinafter simply referred to as CHO (dhfr⁻) cells), mouse L cells, mouse AtT-20, mouse myeloma cells, rat GH3, human FL cells, etc.

Bacteria belonging to the genus Escherichia can be transformed, for edxample, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182 - 187 (1991), Proc. Natl. Acad. Sci. U.S.A., 75, 1929 (1978), etc.

Insect cells or insects can be transformed, for example, according to the method described in Bio/Technology, 6, 47 - 55 (1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263 -267 (1955), published by Shujunsha, or Virology, 52, 456 (1973).

Thus, the transformant transformed with the expression vector containing the DNA encoding the polypeptide can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids [Miller, Journal of Experiments in Molecular Genetics, 431 - 433, Cold Spring Harbor Laboratory, New York, (1972)]. If necessary and desired, a chemical such as 3 β -indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture may be aerated or agitated.

Where yeast is used as the host, the transformant is cultivated, for example, in Burkholder's minimal medium [Bostian, K.L. et al., Proc. Natl. Acad. Sci. U.S.A., 77, 4505 (1980)] or in SD medium supplemented with 0.5% Casamino acids [Bitter, G.A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium [Grace, T.C.C., Nature, 195, 788 (1962)] to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the polypeptide of the present invention can be produced within the cell, in the cell membrane or outside the cell of the transformant.

The polypeptide of the present invention can be separated and purified from the culture described above by the following procedures.

When the polypeptide of the present invention is extracted from the culture or cells, after cultivation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus , the crude extract of the polypeptide of the present invention can be obtained. The buffer used for the procedures may contain a protein modifier such as urea or guanidine hydrochloride, or a surfactant such as Triton X-100™, etc. When the polypeptide is secreted in the culture, after completion of the cultivation the supernatant can be separated from the transformants or cells to collect the supernatant by a publicly known method.

The polypeptide contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc. ; a method utilizing difference in electric charge such as ion exchange chromatography, etc. ; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the polypeptide thus obtained is in a free form, it can be converted into the salt by publicly known methods or modifications thereof. On the other hand, when the polypeptide is obtained in the form of a salt, it can be converted into the free form or in the form of a different salt by publicly known methods or modifications thereof.

The polypeptide produced by the recombinant can be treated, prior to or after the purification, with an appropriate protein modifying enzyme so that the polypeptide can be appropriately modified to partially remove a polypeptide. Examples of the protein-modifying enzyme include trypsin, chymotrypsin, arginyl endopeptidase, protein kinase, glycosidase or the like.

The presence of the thus produced polypeptide of the present invention or salts thereof can be determined by an enzyme immunoassay or Western blotting, or the like using a specific antibody.

Antibodies to the polypeptide of the present invention, or salts thereof may be any of polyclonal antibodies and monoclonal antibodies, as long as they are capable of recognizing the polypeptide of the present invention or its salts thereof.

The antibodies to the polypeptide of the present invention, or salts thereof may be manufactured by publicly known methods for manufacturing antibodies or antisera, using as antigens of the polypeptide of the present invention.

### [Preparation of monoclonal antibody]

### (a) Preparation of monoclonal antibody-producing cells

The polypeptide of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to 6 weeks and 2 to 10 times in total. Examples of the applicable warm-blooded animals are monkeys, rabbits, dogs, guinea pigs, mice, rats, sheep, goats and chicken, with mice and rats being preferred.

In preparation of monoclonal antibody-producing cells, warm-blooded animals, e.g. mice, immunized with an antigen wherein the antibody titer is noted are selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the polypeptide which will be described later, with the antiserum followed by assaying the binding activity of the labeling agent bound to the antibody. The fusion may be operated, for example, by the known Koehler and Milstein method [Nature, 256, 495, 1975]. Examples of the fusion accelerator are polyethylene glycol (PEG), Sendai virus, etc., of which PEG is preferably employed.

Examples of the myeloma cells are NS-1, P3U1, SP2/0 and AP-1, etc., which are the myeloma cells derived from warm-blooded animals. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the polypeptide as an antigen directly or together with a carrier, adding anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid adsorbed with an anti-immunoglobulin antibody or Protein A, adding the polypeptide labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyakku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by publicly known methods, for example, methods applied to separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, by an immunogen (polypeptide antigen) solely, or by preparing a complex of the immunogen and a carrier protein and using it, a warm-blooded animal is immunized in a manner similar to the method described above for the manufacture of monoclonal antibodies. The product containing the antibody to the polypeptide of the present invention or its salts is collected from the immunized animal followed by separation and purification of the antibody.

In the complex of an immunogen and a carrier protein used to immunize a warm-blooded animal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of warm-blooded animals immunized by the method described above.

The polyclonal antibody titer in antiserum can be assayed by the same procedure as that for the determination of antibody titer in the anti-serum described above. The separation and purification of the polyclonal antibody can be carried out, following the method for the separation and purification of immunoglobulins performed as applied to the separation and purification of monoclonal antibodies described hereinabove.

By using the polypeptide of the present invention, human antibody can be prepared in accordance with the method described in Nat Biotechnol. 14, 845 - 851 (1996), Nat Genet 15, 146 - 156 (1997) and PNAS 97(2), 722 - 727 (2000) and the like.

The antisense-DNA containing complementary or substantially complementary base sequence to the DNA encoding the polypeptide of the present invention (in the explanation of antisense-DNA hereinafter, these DNAs are referred to as DNAs of the present invention) may be any antisense-DNA, as far as the DNA contains complementary or substantially complementary base sequence to DNA of the present invention, and further possesses the activity to inhibit expression of the said DNA.

Examples of the substantially complementary base sequence to DNA of the present invention include the base sequences in which all the base sequences are homologous to the complementary base sequence to DNA of the present invention (e.g., complementary chain of DNA of the present invention), or partial base sequence shows homology by more than approximately 70%, preferably more than approximately 80%, more preferably more than approximately 90%, most preferably more than approximately 95%. In particular, the antisense-DNA is preferable, as long as the complementary chain to the base sequence of the part encoding the N-terminus of the polypeptide of the present invention (e.g., base sequence of the initiation codon area, etc.) among the total base sequence of complementary chain of DNA of the present invention, shows homology by more than approximately 70%, preferably more than approximately 80%, more preferably more than approximately 90% and most preferably approximately 95%. These antisense-DNAs can be manufactured by using the DNA synthesizer, which is publicly known.

Due to possessing signal peptide, the polypeptide of the present invention can be secreted efficiently from cells and then exerts important physiological activities as the humoral factor in signal transduction and self-defense.

Hereinafter, explanation is given on the use of the polypeptide or its salts of the present invention (hereinafter, sometimes referred to as the polypeptide of the present invention), DNA encoding the polypeptide of the present invention (hereinafter, sometimes referred to as the DNA of the present invention), antibodies to the polypeptide or its salts of the present invention (hereinafter, sometimes referred to as antibodies of the present invention) and antisense-DNA.
(1) The polypeptide of the present invention, expressing tissue-specifically, can be used as the tissue marker. In other words, the polypeptide of the present invention is useful as the marker for detecting differentiation of tissues, conditions of diseases, and metastasis of cancer. In addition, it can be employed for fractionation of the corresponding receptor, ligand and the binding protein. Furthermore, the polypeptide of the present invention can be used to investigate biological activities in the form of a panel for the publicly known high-through-put screening. Through chromosome mapping, the polypeptide of the present invention can be utilized in studies of genetic diseases.
(2) Prophylactic/therapeutic drugs for various diseases associated with the polypeptide of the present invention

The polypeptide of the present invention exists as the humoral factor in the living body. Therefore, where abnormalities occur in the polypeptide of the present invention or the DNA of the present invention, or they are deficient, or the expressed amount is abnormally decreased or elevated, various diseases, for example, cancer, immunological diseases, respiratory tract diseases, digestive tract diseases, cardiovascular diseases, endocrinological diseases, infections, nervous system diseases and psychological diseases, etc. manifest.

Therefore, the polypeptide and the like of the present invention and the DNA of the present invention can be used as prophylactic/therapeutic agents for various diseases including cancer, immunological diseases, respiratory tract diseases, digestive tract diseases, cardiovascular diseases, endocrinological diseases, infections, nervous system diseases and psychological diseases,etc.

For example, when a patient suffers from inadequate transmission of information in cells, or lack of normal achievement due to decrease or defect of the polypeptide of the present invention in the living body, the intrinsic role of the polypeptide of the present invention, or the like can be adequately or normally expressed in the said patient, through (a) administration of the DNA of the present invention to the said patient, thereby leading to expression of the polypeptide of the present invention and the like in the living body, (b) expression of the polypeptide of the present invention and the like in the cells into which the DNA of the present invention is inserted, followed by transplantation of the cells in question to the patient, or (c) administration of the polypeptide of the present invention and the like to the patient in question.

When the DNA of the present invention is used as the prophylactic/therapeutic agents described above, the DNA itself is administered ; alternatively, the DNA is inserted into an appropriate vector such as retrovirus vector, adenovirus vector, adenovirus-associated virus vector, etc. and then administered to humans or warm-blooded animals in a conventional manner. The DNA of the present invention may also be administered as naked DNA, or formulations containing physiologically acceptable carriers such as adjuvants to assist its uptake by gene gun or through a catheter such as a catheter with a hydrogel.

When the polypeptide of the present invention is used as the prophylactic/therapeutic agents, purified polypeptide with purity of at least 90%, preferably more than 95%, more preferably more than 98%, most preferably more than 99% shall be preferably used.

Examples of the formulations for the polypeptide of the present invention include , if necessary and desired, sugar-coated tablets, capsules, elixirs and microcapsules for oral usage, or parenterally in the form of injectable preparations such as a sterile solution and a suspension in water or with other pharmaceutically acceptable liquid. These preparations can be manufactured by mixing the polypeptide of the present invention with physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum Arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol and polyethylene glycol), a nonionic surfactant (e.g., polysorbate 80^{TH}, HCO-50 or the like), etc. Examples of the oily medium include sesame oil and and soybean oil, which may also be used in combination with a dissolution aid such as benzyl benzoate and benzyl alcohol. The prophylactic/therapeutic agent described above may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared solution for injection is normally filled in an appropriate ampoule.

The vector to which the DNA of the present invention is inserted may also be formulated in the similar manner as described above, and the resultant formulation is usually used parenterally.

Since the thus obtained pharmaceutical preparation is safe and low toxic, the preparation can be administered to humans or animals (e.g., rats, mice, guinea pigs, rabbits, birds, sheep, swine, bovine, horses, cats, dogs, monkeys, etc.).

The dose of the polypeptide of the present invention or the like varies depending on diseases to be treated, subjects to be administered, and routes for administration, ect. ; when the polypeptide of the present invention or the like is orally administered for the purpose of treatment of immunological diseases, the dose to an adult (as 60 kg body weight) is normally about lmg to about 1000 mg, preferably about 10 mg to about 500 mg, more preferably about 10 mg to about 200 mg per day. In parenteral administration, the single dose varies depending on subjects to be administered, target diseases, etc. but it is advantageous, e.g., for treatment of immunological diseases of the adult patient (as 60 kg body weight), to inject the polypeptide in question locally to the lesion in a daily dose of about 1 mg to 1000 mg, preferably about 1 mg to about 200 mg, more preferably about 10 mg to about 100 mg. For other animal species, the corresponding dose as converted per 60 kg body weight can be administered.

### (2) Screening of the candidate compounds as drugs to be used for diseases

Since the polypeptide of the present invention or the like exists as humoral factors in the living body, compounds or their salts to promote functions of the polypeptide of the present invention, etc. can be used as prophylactic/therapeutic drugs for diseases, for example, cancer, immunological dieases, respiratory tract diseases, digestive tract diseases, cardiovascular diseases, endocrinological diseases, infections, nervous system diseases and psychological diseases, etc.

On the other hand, compounds or their salts to inhibit functions of the polypeptide of the present invention, etc. can be used as prophylactic/therapeutic drugs for diseases attributed to overproduction of the polypeptide of the present invention or the like.

Thus, the polypeptide of the present invention, etc. is useful as the reagent for screening of the compounds or their salts which promote or inhibit the functions of the polypeptide of the present invention, etc.

That is, the present invention, provides methods of screening compounds or their salts (hereinafter, sometimes referred to as the stimulant) that promote functions of the polypeptide of the present invention or its salts, or compounds or their salts (hereinafter, sometimes referred to as the inhibitor) that inhibit functions of the polypeptide of the present invention or its salts, characterized by using the polypeptide of the present invention or its salts.

The screening kit of the present invention contains the polypeptide of the present invention or its salts therein.

Examples of the compounds or their salts to be obtained by the screening methods or the screening kit of the present invention include compounds selected among peptides, proteins, non-peptide compounds, synthetic compounds, fermentation products, cell extracts, plant extracts, and animal tissue extracts and plasma, etc and these compounds either promote or inhibit functions of the polypeptide of the present invention or the like.

For the salts of these compounds, similar ones as the salts of the afore-mentioned polypeptide of the present invention are used.

When the compounds thus obtained with use of the screening method or the screening kit are used as the prophylactic/therapcutic agents, they can be processed according to the conventional methods. For example, in the similar manners to be employed for drugs containing the afore-mentioned polypeptide of the present invention, tablets, capusules, elixirs, microcapsules and aseptic solution, and suspensions, etc. can be prepared.

Since the thus obtained pharmaceutical preparation is safe and low toxic, the preparation can be administered to humans or warm-blooded animals (e.g., rats; mice, rabbits, sheep, swine, bovine, horses, birds, cats, dogs, monkeys, etc.).

The dose of the polypeptide of the present invention or its salts varies depending on activity, target diseases, subjects to be administered, and routes for administration, etc.; when a compound which promotes the functions of the polypeptide of the present invention or the like is orally administered for the purpose of treatment of inflammatory diseases, the dose to an adult (as 60 kg body weight) is normally about 0.1 mg to about 100 mg, preferably about 1.0 mg to about 50 mg, more preferably about 1.0 mg to about 20 mg per day. In parenteral administration, the single dose of the said compound varies depending on subject to be administered, target diseases, etc. but it is advantageous, e.g., for treatment of inflammatory diseases or immunological diseases of the adult patient (as 60 kg body weight), to inject intravenously the compound to promote functions of the polypeptide of the present invention, etc. in a daily dose of about 0.01 mg to 30 mg, preferably abut 0.1 mg to about 20 mg, more preferably about 0.1 mg to about 10 mg. For other animal species, the corresponding dose as converted per 60 kg body weight can be administered.

On the other hand, when a compound which inhibits the functions of the polypeptide of the present invention or the like is orally administered, the dose to an adult (as 60 kg body weight) is normally about 0.1 mg to about 100 mg, preferably about 1.0 mg to about 50 mg, more preferably about 1.0 mg to about 20 mg per day. In parenteral administration, the single dose of the said compound varies depending on subject to be administered, target diseases, etc. but it is advantageous, e.g., in case of administration of the compound to promote functions of the polypeptide of the present invention, etc. to an adult (as 60 kg body weight), to administer by intravenous injection at a daily dose of about 0.01 mg to 30 mg, preferably abut 0.1 mg to about 20 mg, more preferably about 0.1 mg to about 10 mg. For other animal species, the corresponding dose as converted per 60 kg body weight can be administered.

### (3) Quantification of the polypeptide or its salts of the present invention

The antibodies to the polypeptide of the present invention, etc. (hereinafter, sometimes referred to as the antibody of the present invention) are capable of specifically recognizing the polypeptide of the present invention, etc. Therefore, the antibodies can be used to quantify the polypeptide of the present invention, etc. in a test fluid, especially for quantification by the sandwich immunoassay.

That is, the present invention provides, for example, the following quantification methods:
(i) a method of quantifying the polypeptide of the present invention and the like in a test fluid, which comprises competitively reacting the antibody of the present invention with the test fluid and a labeled form of the polypeptide of the present invention, and measuring the ratio of the labeled polypeptide of the present invention and the like bound to the antibody; and
(ii) a method of quantifying the polypeptide of the present invention and the like in a test fluid, which comprises reacting the test fluid with the antibody of the present invention immobilized on a carrier and a labeled form of the antibody of the present invention simultaneouosly or sequentially, and measuring the activity of the label on the immobilized carrier.

Using monoclonal antibodies to the polypeptide of the present invention (hereinafter sometimes referred to as the monoclonal antibodies of the present invention), the polypeptide of the present invention can be assayed and also detected by tissue staining or the like. For this purpose, an antibody molecule itself may be used, or F(ab')₂, Fab' or Fab fractions of the antibody molecule may also be used.

Assay methods using antibodies to the polypeptide of the present invention are not particularly limited. Any assay method can be used, so long as the amount of antibody, antigen, or antibody-antigen complex corresponding to the amount of antigen (e.g., the amount of the polypeptide) in the test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H] and [¹⁴C] are used. As the enzyme described above, stable enzymes with high specific activity are preferred ; for example, β-galactosidase, β-glucosidase, alkaline phosphatase, peroxidase, malate dehydrogenase and the like are used. Examples of the fluorescent substance used are fluorescamine and fluorescein isothiocyanate. For the luminescent substance, for example, luminol, luminol derivatives, luciferin, and lucigenin are used. Furthermore, the biotin-avidin system may be used for binding antibody or antigen to the label.

For immobilization of antigen or antibody, physical adsorption may be used. Chemical binding methods conventionally used for insolubilization or immobilization of polypeptides or enzymes may also be used. For the carrier, for example, insoluble polysaccharides such as agarose, dextran, cellulose, etc. ; synthetic resin such as polystyrene, polyacrylamide, silicon, etc., and glass or the like are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the polypeptide of the present invention in the test fluid can be quantified. The order of the primary and secondary reactions may be reversed, and the reactions may be performed simultaneously or with an interval. The methods of labeling and immobilizatin can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the polypeptide of the present invention and the like by the sandwich method, antibodies that bind to different sites of the polypeptide of the present invention are preferably used. That is, in the antibodies used for the primary and secondary reactions, for example, when the antibody used in the secondary reaction recognizes the C-terminal region of the polypeptide of the present invention, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc.

In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody; the immobilized phase is separated from the liquid phase, or antigen in a test fluid and an excess amount of labeled antibody are reacted; immobilized antigen is then added to bind the unreacted labeld antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the polypeptide of the present invention are constructed by adding the usual technical consideration in the art to the conventional conditions and procedures. For the details of these general technical means, reference can be made to the following reviews and texts.

For example, followings can be referred to : Hiroshi Irie, ed. "Radioimmunoassay" (Kodansha, published in 1974), Hiroshi Irie, ed. "Sequel to the Radioimmunoasay" (Kodansha, published in 1979), Eiji Ishikawa, et al. ed. "Enzyme immunoassay" (Igakushoin, published in 1978), Eiji Ishikawa, et al. ed. "Immunoenzyme assay" (2^{nd} ed.) (Igakkushoin published in 1982), Eiji Ishikawa, et al. ed. "Immunoenzyme assay" (3^{rd} ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the polypeptide of the present invention or the like can be quantified with high sensitivity, using the antibodies of the present invention.

When the concentration of the polypeptide of the present invention is found to be reduced by means of quantifying the polypeptide of the present invention or the like in vivo using the antibodies of the present invention, diagnosis can be made on various diseases, for example, cancer, immunological diseases, respiratory tract diseases, digestive tract diseases, cardiovascular diseases, endocrinological diseases, infections, nervous system diseases and psychological diseases, etc., or on highly predisposing conditions leading to onset in future.

The antibodies of the present invention can also be used for specifically detecting the polypeptide of the present invention existing in test samples such as body fluids or tissues. The antibodies may also be used for preparation of antibody columns for purification of the polypeptide of the present invention, for detection of the polypeptide of the present invention in each fraction upon purification, and for analysis of the behavior of the polypeptide of the present invention in the test cells.

### (4) Gene diagnostic agent

By using the DNA of the present invention as a probe, an abnormality (gene abnormality) of the DNA or mRNA encoding the polypeptide of the present invention in humans or warm-blooded animals (e.g., rats, mice, guinea pigs, rabbits, birds, sheep, swine, bovine, horses, cats, dogs, and monkeys etc.) can be detected. Therefore, the DNA of the present invention is useful as a gene diagnostic agent for the damage against the DNA or mRNA, its mutation, or its decreased expression, or increased expression or overexpression of the DNA or mRNA.

The gene diagnosis described above using the DNA of the present invention can be performed by, for example, the publicly known Northern hybridization assay or the PCR-SSCP assay [Genomics, 5, 874 - 879 (1989) ; Proceedings of the National Academy of Sciences of the United States of America, 86, 2766 - 2770 (1989)].

For example, when decreased expression, and mutation of DNA are detected by Northern hybridization assay and PCR-SSCP assay, respectively, diagnosis can be made on higher morbidity of diseases including, for example, cancer, immunological diseases, respiratory tract diseases, digestive tract diseases, cardiovascular diseases, endocrinological diseases, infections, nervous system diseases and psychological diseases, etc.

### (5) Drugs containing antisense-DNA

The antisense-DNA can complementarily bind to the DNA of the present invention, thereby inhibiting expression of the DNA in question. Therefore, since the antisense-DNA can inhibit functions of the polypeptide of the present invention, etc. and the DNA of the present invention in vivo, for example, the antisense-DNA can be used as a prophylactic/ therapeutic agent for diseases due to overexpression of the polypeptide of the present invention or the like.

When the above-cited antisense-DNA is employed as a prophylactic/therapeutic agent as described above, the same procedures as those employed for a prophylactic/therapeutic agent containing the DNA of the present invention as stated above for various diseases can be applied..

For example, when the antisense-DNA of the present invention is used as the prophylactic/therapeutic agents described above, the antisense-DNA itself is administered ; alternatively, the antisense-DNA is inserted into an appropriate vector such as retrovirus vector, adenovirus vector, adenovirus-associated virus vector, etc. and then administered in a conventional manner. The antisense-DNA of the present invention may also be administered as naked antisense-DNA, or formulations containing physiologically acceptable carriers such as adjuvants to assist its uptake by gene gun or through a catheter such as a catheter with a hydrogel.

Furthermore, the antisense-DNA can be used as a diagnostic oligonucleotide probe to investigate presence and/or expression of the DNA of the present invention in tissues and cells.

### (6) Pharmaceutical components containing the antibody of the present invention

The antibody of the present invention possessing neutralizing effect against the activity of the polypeptide of the present invention etc. can be used as a prophylactic/ therapeutic agent for diseases due to overexpression of the polypeptide of the present invention or the like.

The prophylactic/therapeutic agent containing the antibody of the present invention for the above stated diseases can be used orally in the form of liquid preparation, or parenterally as a component of appropriate preparations, to humans or mammalians (e.g., rats, rabbits, sheep, swine, bovine, cats, dogs, monkeys, etc.). The dose of the antibody of the present invention varies depending on subjects to be administered, diseases to be treated, symptoms, and routes for administration, etc.; it is favorable to select the single dose of the antibody of the present invention at normally about 0.01 mg to about 20 mg/kg of body weight, preferably about 0.1 mg to about 10 mg/kg of body weight, more preferably about 0.1 mg to about 5 mg/kg of body weight, once to 5 times per day, preferably once to 3 times per day via intravenous injection. In parenteral and oral administrations, the dose corresponding to the above can be administered. In case with severe symptoms, the dose may be increased according to the symptoms.

The antibody of the present invention can be administered as it is, or as an appropriate component for drugs. The pharmaceutical composition to be administered as described above contains pharmaceutically acceptable carrier, diluents or vehicles to the antibody of the present invention or its salts. The composition can be provided in the pharmaceutical form applicable to oral or parenteral administration.

That is, examples of the composition include solid or liquid preparation, in more details, tablets (including sugar coated tablets, and film-coated tablets), pills, granules, powder forms, capsules (including soft capsules) , syrups, emulsions, suspensions, etc. The composition is manufactured according to the publicly known methods and contains carrier, diluents or vehicles, which are usually used in the pharmaceutical field. Examples of the component for carriers and vehicles for tablets include lactose, starch, sucrose and magnesium stearate, etc.

As the composition for parenteral administration, for example, injectable forms and suppositories are used; for injectable forms, subcutaneous, intracutaneous, intramuscular injections and infusion are included. The above-stated injectable preparations can be prepared in accordance with the publicly known methods, for example, through dissolution, suspension or emulsification of the above-described antibody or its salts in sterile aqueous solution or oily liquid usually applicable to preparation of injectable preparations. Examples of an aqueous medium for injection include physiological saline and an isotonic solution containing glucose and other auxiliary agents and may be used in combination with an appropriate dissolution aid such as an alcohol (e.g., ethanol) , a polyalcohol (e.g., propylene glycol and polyethylene glycol), a nonionic surfactant [e.g., polysorbate 80^{TH}, HCO-50 (polyoxyethylene (50 mol) adduct of hydrogenated caster oil)]. Examples of the oily medium include sesame oil and soybean oil, which may also be used in combination with a dissolution aid such as benzyl benzoate and benzyl alcohol. The thus-prepared solution for injection is normally filled in an appropriate ampoule. The suppository to be used for rectal admiministration is prepared by mixing the above-stated antibody or its salts with the conventional vehicle for suppositories.

It is advantageous to prepare the pharmaceutical preparation of the above-stated medical composition for oral or parenteral use in a unit dosage form meeting the dose of the active ingredient. Examples of the formulations in the administration unit include tablets, pills, capsules, injectable preparations (ampoules) and suppositories, etc. It is preferable to contain usually 5 mg to 500 mg of the above-stated antibody per unit preparation, in particular, 5 mg to 100 mg for injectable preparation and 10 mg to 250 mg for other preparation forms.

Incidentally, the above-stated respective compositions may contain other active ingredient, as far as combination with the above-stated antibody would not result in unfavorable interactions.

### (7) DNA-transferred animals

The present invention provides non-human mammals carrying the extrinsic DNA encoding polypeptide of the present invention, etc. (hereinafter abbreviated as extrinsic DNA of the present invention) and the variant DNA (hereinafter sometimes referred to as extrinsic variant DNA).

That is, the present invention provides the followings:
(1) Non-human mammals carrying the extrinsic DNA of the present invention and its variant DNA.
(2) Animals described in the above (1), which the non-human mammals are rodents.
(3) Animals descried in the above (2), which the rodents are mice or rats, and
(4) Recombinant vector carrying either the extrinsic DNA of the present invention or its variant DNA, which can be expressed in mammals.

Non-human mammals carrying either the extrinsic DNA of the present invention or its variant DNA (hereinafter, abbreviated as the DNA-transferred animals) can be prepared by transferring the target DNA using calcium phosphate method, electric pulse method, lipofection method, coagulation method, micro-injection method, particle gun method, DEAE-dextran method, etc. to non-fertilized ovum, fertilized ovum, spermatozoon, and embryo cells including its spermatogonium, preferably at the stage of embryo genesis during the genesis of non-human mammals (more preferably at the unicellular stage or at the stage of fertilized ova, and also before octacellular stage in general). The DNA transfer method can transfer the extrinsic DNA of the present invention into somatic cells, organs and tissue cells, thereby utilizing them for cell culture and tissue culture, and further, the DNA-transferred animals of the present invention can be prepared by fusing these cells with the above-cited embryo cells by the publicly known cell fusion method.

Examples of the non-human mammals include, for example, bovine, swine, sheep , goats, rabbits, dogs, cats, guinea pigs, hamsters, mice, rats and the like. Among them, from the view-point of preparation of disease-affected animal models, rodents, it is preferable to use rodents which have relatively shorter period of ontogeny and biological cycles and are easily raised, in particular to use mice (e.g., as the pure line, C57BL/5 line, DBA2 line, BALB/c line, ICR line, etc.) or rats (e.g., Wistar, SD rats, etc.).

The "mammals" for the variant vectors which can expression mammals, include humans, in addition to above-stated non-human mammals.

The extrinsic DNA of the present invention does not mean the DNA of the present invention which is intrinsically possessed by non-human mammals, but the DNA of the present invention which is once isolated and extracted from mammals.

Examples of the variant DNA of the present invention include the DNA with a certain variation (e.g., mutation) within the base sequence in the original DNA of the present invention, in more details, the DNA with addition of base, defect of base, and replacement with other base; abnormal DNA is also included.

The above-stated abnormal DNA stands for the DNA expressing the abnormal polypeptide of the present invention ; for example, the DNA expressing the polypeptide which inhibits functions of the normal polypeptide of the present invention is employed.

The extrinsic DNA of the present invention is any of the DNA derived from either homogeneous or heterogeneous mammals to the target animal. When the DNA of the present invention is transferred to the target animal, it is generally advantageous to use the DNA in a gene construct ligated downstream of a promoter that can express the DNA in animal cells. For example, when the human DNA of the present invention is transferred, e.g., the DNA construct (e.g., vector, etc.), in which the human DNA of the present invention is ligated downstream of various promoters that can express the DNA derived from various mammals (e.g., rabbits, dogs, cats, guinea pigs, hamsters, rats, mice or the like) containing the DNA of the present invention highly homologous to the human DNA, is microinjected to mouse fertilized ova; thus, the DNA-transferred mammal, which is capable of producing a high level of the DNA of the present invention, can be produced.

Examples of the expression vector for the polypeptide of the present invention include plasmid derived from Escherichia coli, plasmid derived from Bacillus subtilis, plasmid derived from yeast, bacteriophage such as λ phase, retrovirus including Moloney's leukemia virus, animal viruses such as vaccinia virus or baculovirus, etc. Among them, plasmid derived from Escherichia coli, plasmid derived from Bacillus subtilis or plasmid derived from yeast are preferably employed.

Examples of the promoters that can regulate the DNA expression include ① the DNA promoters derived from virus (e.g., simian virus, cytomegalovirus, Moloney's leukemia virus, JC virus, mammalian cancer virus, poliovirus, etc.), ② the promoters derived from various mammals (humans, rabbits, dogs, cats, guinea pigs, hamsters, rats, mice, etc.), for example, albumin, insulin II, uropulaquin II, elastase, erythropoietin, endothelin, myocreatinkinase, glia fiber acidic protein, glutathione-S-transferase, platelet-derived growth factor β, keratin-Kl, K10 and K14, collagen type I and type II, cyclic AMP dependent proteinkinase β1 subunit, dystrophin, tartaric acid resistant alkaline phosphatase, atrium sodium diuretic factor, endothelial receptor tyrosine kinase (usually abbreviated as Tie2), sodium potassium adenosin triphosphate oxidation enzyme (Na, K-ATPase),neurofilament light chain, metallothionein I and IIA, metalloproteinase 1 tissue inhibitor, MHC class I antigen (H-2L), H-ras, rennin, dopamine β-hydroxylation enzyme, thyroid peroxidase (TPO), polypetide chain elongation factor 1α (EF-1 α), β actin, α and β myosin heavy chain, myosin light chain 1 and 2, myelin basic protein, thyroglobulin, Thy-1, immunoglobulin, H chain variable part (VNP), serum amyloid P component, myoglobin, troponin C, smooth muscle α actin, preproenkephalin A, and vasopressin, etc. Among them, it is advantagenous to use cytomegalovirus promoter capable of highly expressing in the whole body, human polypeptide chain elongation factor 1 α (EF-1 α) promoter, human and chicken β actin promoter, etc.

The above-cited vectors are preferably to possess the sequence (generally, referred to as terminator) to terminate transcription of the target messenger RNA in DNA transferred mammals ; for example, examples of such terminators include the one allowing use of the sequence of respective DNA derived from virus and various mammals, preferably SV40 terminator of simian virus.

Furthermore, for the purpose of highly expressing the target extrinsic DNA, it is feasible to ligate the splicing signal, enhancer region and a part of intron of eukaryote DNA either upstream a promoter region, between the promoter region and translation region, or downstream the translation region, depending upon the objective.

The said translation region can be prepared by conventional DNA technological methods in which the DNA construct that can express in the transferred animals is ligated downstream the above-stated promoter, or if necessary and desired, upstream the transfer termination region according to the conventional DNA technological procedures.

The transfer of the extrinsic DNA of the present invention at the fertilized egg cell secures the presence of the DNA in all germ and somatic cells in the produced mammals. The presence of the extrinsic DNA of the present invention in the germ cells in the DNA-transferred animal means that all germ and somatic cells contain the extrinsic DNA of the present invention in all progenies of the animal. The progenies of the animal that took over the gene contain the extrinsic DNA of the present invention in all germ and somatic cells.

The non-human mammals with extrinsic and normal DNA of the present invention being transferred can be maintained and bred in the conventional environment as the animals carrying the DNA of the present invention after confirming the stable retention of the gene in the animals through mating.

The transfer of the extrinsic DNA of the present invention at the fertilized egg cell secures the excessive presence of the DNA in all germ and somatic cells in the produced mammals. The excessive presence of the extrinsic DNA of the present invention in the germ cells in the DNA-transferred animal means that all germ and somatic cells contain excessive amount of the extrinsic DNA of the present invention in all progenies of the animal. The progenies of the animal that took over the gene contain excessive amount of the extrinsic DNA of the present invention in all germ and somatic cells.

Furthermore, mating male and female animals containing the objective DNA results in acquiring homozygote animals having the transferred DNA on both homologous chromosomes.

Since the normal DNA of the present invention is highly expressed in the non human mammals in which the normal DNA of the present invention is carried, the animals can be used as the pathological model animals because stimulation of functions of the intrinsic normal DNA finally results in manifestation of hyperergasia of the polypeptide of the present invention. For example, it is possible to clarify the pathological mechanism of hyperergasia of diseases associated with polypeptides of the present invention and to investigate therapeutic methods of these diseases, by using the normal DNA-transferred animals of the present invention.

In addition, the mammals in which the extrinsic normal DNA of the present invention has been transferred can also be used for screening tests of therapeutic drugs of the diseases associated with the polypeptide of the present invention because the transferred animals present with symptoms attributable to increase in the liberated polypeptide of the present invention.

The non-human mammals with extrinsic abnormal DNA of the present invention being transferred can be maintained and bred in the conventional environment as the animals carrying the DNA of the present invention after confirming the stable retention of the gene in the animals through mating. Furthermore, the target extrinsic DNA is transferred into the aforementioned plasmid and then, used as the source for tissue culture. The DNA construct with the promoter can be prepared by the conventional DNA technological procedures. The transfer of the abnormal DNA of the present invention at the fertilized egg cell secures the presence of the DNA in all germ and somatic cells in the produced mammals. The presence of the abnormal DNA of the present invention in the germ cells in the DNA-transferred animal means that all germ and somatic cells contain the abnormal DNA of the present invention in all progenies of the animal. The progenies of the animal that took over the gene contain the abnormal DNA of the present invention in all germ and somatic cells. Homozygote animals having the transferred DNA on both homologous chromosomes are acquired whereby mating male and female animals thus obtained can be bred to obtain the progenies having the target DNA.

Since the abnormal DNA of the present invention is highly expressed in the non-human mammals carrying the abnormal DNA of the present invention, inhibition of functions of the intrinsic normal DNA finally results in manifestation of the function -inactivated non-responder to the polypeptide of the present invention; therefore, it can be used as the pathological model animal. For example, it is possible to clarify the pathological mechanism of the function-inactivated non-responder to the polypeptide of the present invention and to investigate therapeutic methods of this disease, by using the abnormal DNA-transferred animals of the present invention.

As the specific applicability, the animals highly expressing the abnormal DNA of the present invention can be the model animals to be used for investigation of the mechanism involved in the function disorders (dominant negative effect) of the normal polypeptide by the abnormal polypeptide of the present invention in the function-inactivated non-responder to the polypeptide of the present invention.

Since the mammals in which the abnormal extrinsic DNA of the present invention has been transferred present with symptoms associated with increase of the liberated polypeptide of the present invention, these animals can also be used for screening tests of therapeutic drugs of the function-inactivated non-responder to the polypeptide of the present invention.

In addition, other applicability of the above-cited 2 kinds of the DNA-transferred animals of the present invention include the followings:
① Use as the cell source for tissue culture.
②Analysis of the relationship with the polypeptide to be specifically expressed or activated by the polypeptide of the present invention, by means of direct analysis of DNA or RNA in tissues of the DNA-transferred animals of the present invention, or analysis of the polypeptide tissues which are expressed by DNA.
③ Study on function of cells derived from tissues known to be hardly cultured, based on the usage or the cultured cells carrying the DNA by the standard tissue culture technology.
④ Screening of the drug candidates which are capable to potentiate functions of the cells by using the cells described above in ③, and
⑤ Isolation and purification of the variant polypeptide of the present invention and preparation of its antibody.

Using the DNA-transferred animals of the present invention, for example, the clinical symptoms due to function-inactivated non-responder to the polypeptide of the present invention and other diseases associated with the polypeptide of the present invention can be investigated. Furthermore, using these animals, more detailed pathological findings in respective organs of the disease models related to the polypeptide of the present invention can be obtained, thereby contributing to development of brand-new therapeutic remedies, furthermore, to research and treatment of secondary diseases of the said disease.

Respective organs are isolated from the DNA-transferred animals of the present invention, cut into small pieces, treated with polypeptide degrading enzyme such as trypsin, and then the liberated DNA-transferred cells are obtained; subsequently, it is possible to perform phylogenetic analysis of its culture or cultured cells. These organs permit identification of the polypeptide producing cells of the present invention, and investigation of correlation with apoptosis, differentiation or growth, mechanism of the signal transduction and further study on abnormality of them, thereby being used as useful materials for research for the polypeptide of the present invention and clarification of its mechanism of action.

Using the afore-mentioned test methods and quantification methods, it is possible to provide the efficient and quick screening method for therapeutic drugs of the diseases, whereby development of therapeutic drugs for the diseases associated with the polypeptide of the present invention, including function-inactivated non-responder to the polypeptide of the present invention by utilizing the DNA-transferred animal of the present invention. In addition, using the DNA-transferred animals of the present invention or the extrinsic DNA expressing vector of the present invention, it is possible to investigate and develop the DNA therapeutic method to treat diseases associated with the polypeptide of the present invention.

### (8) Knock-out animals

The present invention provides the non-human mammal embryo stem cells in which the DNA of the present invention has been inactivated, and the non-human mammals with the inadequate expression of the DNA of the present invention.

That is, the present invention provides:
(1) Non-human mammal embryo stem cells in which the DNA of the present invention has been inactivated.
(2) The embryo stem cells as described in the above (1), in which the DNA has been inactivated by insertion of the reporter gene (e.g., β galactosidase gene derived from E. coli).
(3) The embryo stem cells as described in the above (1), which is resistant to neomycin.
(4) The embryo stem cells as described in the above (1), where rodents represent the non-human mammals.
(5) The embryo stem cells as described in the above (1), where rodents represent mice.
(6) The DNA insufficiently expressing non-human mammals in which the DNA of the present invention has been inactivated.
(7) The non-human mammals as described in the above (6), in which the DNA has been inactivated by insertion of the reporter gene (e.g., β galactosidase gene derived from Escherichia coli) and the reporter gene can express under regulation of the promoter to the DNA of the present invention.
(8) The non-human mammals as described in (6), where the non-human mammals are rodents.
(9) The non-human mammals as described in (8), where the rodents are mice, and
(10) The screening methods for the compounds or their salts that either promote or inhibit the promoter activity to the DNA of the present invention, characterized by detecting the expressed reporter gene following administration of the test compounds to the following cells or animals as described in (7).

Artificial variation is applied to the DNA of the present invention that is carried by the non-human mammals to inhibit the expression ability of the DNA or to actually eliminate the activity of the polypeptide of the present invention encoded by the DNA; then the obtained non-human mammal's embryo stem cells (hereinafter abbreviated as ES cells) in which the DNA has actually no expressing ability of the polypeptide of the present invention (hereinafter sometimes referred to as knocked-out DNA) are called as the non-human mammal's embryo stem cells in which the DNA of the present invention is inactivated.

As the non-human mammals, the same animals as stated above can be employed.

Examples of the methods to apply artificially variation to the DNA of the present invention include deletion of partial or the whole DNA of the present invention, insertion of other DNA or replacement with other DNA. The knocked-out DNA of the present invention may be prepared by these variations, for example, staggering the reading frames of the codon, or destruction of function of the promoter or the exon.

As actual examples, the non-human mammal's embryo stem cells in which the DNA of the present invention has been inactivated (hereinafter, abbreviated as either the DNA inactivated ES cells of the present invention or the knock-out ES cells of the present invention) can be obtained by selecting the knock-out ES cells of the present invention through the following methods ; that is, ①the DNA of the present invention existing in the target non-human mammals is isolated, and to the exon region, drug resistant gene represented by neomycin resistant gene, hygromycin resistant gene, or the reporter genes represented by lacZ (β-galactosidase gene), cat (chloramphenicol acetyltransferase gene) , etc. are inserted to destruct the functions of the exon, or ② the DNA sequence terminating the transcription of the gene (e.g., poly A addition signals, etc.) is inserted to the intron region between the exons to eliminate the synthetic ability of complete messenger RNA; and subsequently, the DNA chain carrying the DNA sequence to destruct the gene (hereinafter abbreviated as the targeting vector) is inserted into the chromosomes of the animal by, for example, the homologous recombination method. Then, the resultant ES cells are analyzed by the Southern hybridization analysis by using the DNA of the present invention or the near-by DNA sequence as the probe, or the PCR method with use of the DNA sequence on the targeting vector and the near-by DNA sequence except for the DNA of the invention which has been used for preparation of the targeting vector, as the probe.

The ES cells already established as stated above may be subjected to inactivation of the DNA of the present invention by homologous recombination, etc. and alternatively, the newly established ES cells in accordance with the publicly known Evans and Kaufman's method may also be used. In the case of ES cells from mice, for example, the ES cells of 129 strain is generally employed; however, since the immunological background is unclear, due to clearness of immunological and genetic background, BDF₁ mice (F₁ of C57BL/6 and DBA/2) that is produced by cross between DBA/2 with C57BL/6 mice or C57BL/6 and is characterized with improved number of ovulation may also be favorably used. In addition to advantages endowed to BDF1 mice such as large number of ovulation and tough nature of eggs, BDF₁ mice has C57BL/6 mice as the background ; furthermore, when the pathological model mice is generated, they can be advantageously used in that the hereditary backgrounds of the ES cells obtained from BDF₁ mice can be replaced with C57BL/6 mice following backcross with C57BL/6 mice.

When ES cells are established, blastcyst at 3.5 days after fertilization is generally used, but except for this, eggs under eight cell stage are collected and then cultured, leading to efficient collection of large number of initial embryo through culture up to the stage of blastcyst.

ES cells of both sexes can be used, but male ES cells are more favorable in preparation of genital chimera. It is also desirable to make judgement of male or female at early stages as possible for the benefit of reducing unnecessary labors involved in laborious cultures.

One example of judgement whether ES cells are male or female consists of amplification and detection of the gene located in the sex determining region on Y chromosome by PCR method. This method requires approximately as small as one colony of ES cells (about 50 cells) in sharp contrast with approximately 10⁶ cells being required for nuclear type analysis, permitting the initial selection of ES cells at the early stage of culture by means of judgment of male or female, together with remarkable reduction of labors at the early stage of culture due to possible selection of male cells even at an early stage.

The second selection can be conducted by confirmation of the chromosome number based on G-Banding method. The number of chromosome to be obtained from the ES cells is expected to be 100%, if possible ; however, when it is difficult to secure 100% due to physical procedures etc., upon establishment, it is desirable to knock out the gene of the ES cells and then to make cloning to the normal cells (e.g., the cells with the number of chromosome; 2n=40 in the case of mice) again.

Although the embryo stem cell strain thus obtained usually demonstrates excellent multiplication, this strain tends to lose its ability of ontogeny, indicating necessity of careful subcultures. For example, this strain is to be cultured at about 37°C on the appropriate feeder cells such as STO fibroblast cells in CO₂ incubator (preferably, 5% CO₂, 95% air or 5% O₂, 5% CO₂ and 90% air) in the presence of LBF (1 - 10000U/ml) ; subsequently, upon subcultures, the strain shall be treated with trypsin/EDTA solution (usually, 0.001 - 0.5% trypsin/0.1 - 5mM EDTA, preferably approximately 0.1% trypsin/lmM EDTA) to obtain single cells, which are charged on the freshly prepared feeder cells. These subcultures are usually performed every 1 - 3 days. On such occasions, cells are microscopically observed and when any morphologically abnormal cells are detected, it is desirable to discard this culture cell.

When the ES cells are subjected to monolayer culture up to the higher density under appropriate conditions, or suspension culture until cellular mass is formed, the ES cells can be differentiated to become various types of cells including parietal muscle, visceral muscle and myocardium [M. J. Evans and M. H. Kaufman, Nature, vol 292, 154, 1981; G. R. Martin Proc. Natl. Acad. Sci. U.S.A. vol 78, 7634, 1981 ; T.C. Doetschman et al., Journal of Embryology and Experimental Morphology, vol 87, 27, 1985]. The DNA inadequately-expressing cells that are obtained from the ES cells of the present invention by differentiation are useful in cellular biological investigations of polypeptide of the present invention.

By relatively comparing the expression through determination of mRNA amount of the animal by the publicly known procedures, the non-human mammals with inadequately expressing DNA of the present invention can be distinguished from normal animals.

As the non-human mammals, the same animals as stated above can be employed.

Where the targeting vector as prepared stated above is introduced into mouse embryo stem cells or mouse egg cells and after introduction, the DNA sequence of the inactivated DNA of the present invention in the targeting vector can replace the DNA of the present invention on chromosome of mouse embryo stem cells or mouse egg cells, by the so called homologous recombination, the DNA of the present invention can be knocked-out in the DNA inadequately expressing non-human mammals of the present invention.

The cells with the DNA of the present invention being knocked out can be detected by either Southern hybridization using the DNA sequence of the present invention or the nearby DNA sequence as the probe, or the PCR method analysis with use of the DNA sequence on the targeting vector and the nearby DNA sequence as the primer ; in the meantime, the nearby DNA sequence stands for the DNA sequence except for the DNA of the present invention that is derived from mice and used as the targeting vector. When the non-human mammal embryo stem cells are used, homologous recombination of the gene is conducted to perform cloning of the cell strains having the inactivated DNA of the present invention and then, at the appropriate time, for example, these cells are infused into non-human mammal embryo or blastcyst at the eight cells stage, followed by transplantation of the thus prepared chimera embryo into the uterus of pseudo-pregnant non-human mammals. The resultant animal is a chimera animal comprising the cells with the normal DNA locus of the present invention and the cells with the artificially mutated DNA locus of the present invention.

When a part of the reproductive cells of the chimera animals possesses the partially mutated DNA locus of the present invention, the animal comprising the cells in all the tissues, carrying the artificially mutated DNA locus of the present invention can be obtained by selection, for example, with coat color judgment from the animal groups that are obtained by mating of the chimera animal with the normal animal. Usually, the animal thus obtained presents with inadequate hetero expression of the polypeptide of the present invention; therefore, mutual mating of the animal with the inadequate hetero expression of the polypeptide of the present invention and subsequent selection of F₁ offsprings leads to obtaining the animals with the inadequate homo expression of the polypeptide of the present invention.

In case of using egg cells, for example, transgenic non-human mammals carrying the targeting vector in the chromosome can be obtained by infusion of the DNA solution into the nucleus of the egg cell by microinjection method. Further, in comparison with the transgenic non-human mammals, the mammals having the mutation in the DNA locus of the present invention through the genetic homologous recombination can be obtained.

After confirmation that the animal obtained by mating has the knocked-out DNA, the animals having the knocked-out DNA of the present invention can be bred and subcultured under the conventional breeding conditions.

Furthermore, the conventional methods are applicable to establishment and maintenance of the germ line. In more details, mating of the animals of both sexes carrying the inactivated DNA can produce the homozygous animals that have the inactivated DNA on both homologous chromosomes. The homozygous animal can be efficiently obtained by raising under the condition comprising the following combination ratio of animals such as a normal animal and plural number of homozygous animals against a dam. By mating of males and females of heterozygous animals, the homozygous animals and the heterozygous animals having the inactivated DNA can be bred and subcultured.

The non-human mammal's embryonic stem cells containing the inactivated DNA of the present invention are remarkably useful when the non-human mammals with the inadequately expressing DNA of the present invention are prepared.

Since the non-human mammals with the inadequately expressing DNA of the present invention are devoid of various biological activities which can be induced by the polypeptide of the present invention, these animals are qualified to be the animal models presenting with these diseases that are caused by inactivation of the biological activities of the present polypeptide of the present invention; therefore, they can be useful in investigation of pathological studies and therapeutic methods of these diseases.

### (8a) Screening methods for the compounds that exert the prophylactic/therapeutic effects against diseases attributable to deletion and lesion of the DNA of the present invention.

The non-human mammals with the inadequately expressing DNA of the present invention can be used for screening of the compounds that have the prophylactic/therapeutic effects against the diseases (e.g., cancer, immunological diseases, respiratory tract diseases, digestive tract diseases, cardiovascular diseases, endocrinological diseases, infections, nervous system diseases and psychological diseases, etc.) attributable to deletion and lesion of the DNA of the present invention.

That is, the present invention provides the screening methods of the compounds or their salts that have the prophylactic/therapeutic effects against diseases caused by the deletion and lesion of the DNA of the present invention, following administration of the test compounds to the non-human mammals with the inadequately expressing DNA of the present invention and then observation/determination of the changes of the animals in question.

As the non-human mammals with the inadequately expressing DNA of the present invention that are used in the above-stated screening methods, the same as those afore-mentioned can be referred to.

Examples of the test compounds include peptides, proteins, non-peptide compounds, synthetic compounds, fermentation products, cell extracts, plant extracts, and animal tissue extracts and plasma, etc., but these compounds are any of new compounds or the publicly known compounds.

In detail, the non-human mammals with the inadequately expressing DNA of the present invention are treated with the test compounds and they are compared with the intact reference animals, whereby the prophylactic/therapeutic effects of the test compounds can be assayed with use of changes in respective organs, tissues of the animal and symptoms of the diseases as the index.

Examples of the treatment methods of the test animals with the test compounds include oral administration and intravenous injection, but they can be properly selected depending on the symptoms of the test animals and properties of the test compounds. The dosage of the test compound can be properly selected according to profiles of the test compounds.

When the compound possessing the prophylactic/therapeutic effects against pancreas dysfunction is to be screened, for example, glucose loading treatment is conducted to the non-human mammals with the inadequately expressing DNA of the present invention and after administration of the test compound before or after glucose loading, the time-course change of blood sugar levels and body weights of the animals is determined.

The compounds to be obtained through the screening of the present invention are those compounds selected from the above-stated test compounds. Since they have the prophylactic/therapeutic effects against the diseases (e.g., cancer, immunological diseases, respiratory tract diseases, digestive tract diseases, cardiovascular diseases, endocrinological diseases, infections, nervous system diseases and psychological diseases, etc.) attributable to deletion and lesion of the polypeptide of the present invention, they can be used as the drugs characteristic of safe and low toxic prophylactic/therapeutic agents against these diseases. Furthermore, the compounds derivatized from the compounds obtained by the above-stated screening can be also employed in the similar manner.

The compounds obtained by the screening may be used in their salt's forms. As the salts of these compounds, the salts with physiologically acceptable acids (e.g., inorganic and organic acids) and bases (e.g., alkaline metals) may be used, in particular salts with physiologically acceptable acids being preferable. Examples of such salts include salts with inorganic acids (e.g., hydrochloric acid, phosphoric acid, hydrogen bromide, sulfuric acid), or organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The pharmaceuticals comprising the compounds and their salts obtained by the screening method can be manufactured in the similar manners as those for the drugs comprising the polypeptide of the present invention as described above.

Since the thus obtained pharmaceutical preparation is safe and low toxic, the preparation can be administered to humans or mammals (e.g., rats, mice, guinea pigs, rabbits, sheep, swine, bovine, horses, cats, dogs, monkeys, etc.).

The doses of the compounds or their salts of the present invention vary depending on target diseases, subjects to be administered, and routes for administration, etc. ; when a compound is orally administered for the purpose of treatment of inflammatory diseases, the dose to an adult (as 60 kg body weight) is normally about 0.1mg to about 100 mg, preferably about 1.0 mg to about 50 mg, more preferably about 1.0 mg to about 20 mg per day. In parenteral administration, the single dose of the said compound varies depending on subjects to be administered, target diseases, etc. but it is advantageous, e.g., for treatment of inflammatory diseases of the adult patient (as 60 kg body weight), to inject intravenously the compound in a daily dose of about 0.01 mg to 30 mg, preferably abut 0.1 mg to about 20 mg, more preferably about 0.1 mg to about 10 mg. For other animal species, the corresponding dose as converted per 60 kg body weight can be administered.

### (8b) Screening methods for the compounds that promote or inhibit the activity of the promoter against the DNA of the present invention.

The present invention provides the screening method for the compound or its salts that promote or inhibit the activity of the promoter against the DNA of the present invention, characterized by detecting expression of the reporter gene after administration of the test compound to the non-human mammals with the inadequately expressing DNA of the present invention.

In the above-stated screening method, the non-human mammals with the inadequately expressing DNA of the present invention, to be employed are the non-human mammals with the inadequately expressing DNA of the present invention in which the DNA of the present invention is inactivated by introduction of the reporter gene, together with the reporter gene being able to express under regulation of the promoter against the DNA of the present invention.

As the test compound, the same as stated above are included.

As the reporter gene, the same as stated above can be employed and it is advantageous to use β galactosidase gene (1acZ), soluble alkaline phosphatase gene or luciferase gene.

In the non-human mammals with the inadequately expressing DNA of the present invention in which the DNA of the present invention is replaced with the reporter gene, tracing the expression of the substance encoded by the reporter gene permits detection of activities of the promoter because the reporter gene exists under the regulation of the promoter against the DNA of the present invention.

For example, when a part of the DNA region encoding the polypeptide of the present invention is replaced with the β galactosidase gene (1acZ) derived from Escherichia coli, β galactosidase gene is expressed instead of the polypeptide of the present invention, in the tissues where the polypeptide of the present invention is expressed in situ. Accordingly, when staining is performed with use of the reagent such as 5-bromo-4-chloro-3-indoryl-β-galactopiranoside (X-gal) known to be the substrate of β-galactosidase, expression status of the polypeptide of the present invention can be easily observed in vivo. In more details, the mice with defect of the polypeptide of the present invention or its tissue section is fixed in glutalaldehyde, washed with phosphate-buffered physiological saline (PBS), treated with the dye solution containing X-gal at room temperature or 37°C for approximately 30 minutes or 1 hour, followed by rinsing the tissue specimen with 1mM EDTA/PBS solution to stop the β galactosidase reaction for observation of the color reaction. Alternatively, mRNA encoding 1acZ may be detected according to the conventional method.

The compound and its salts that are obtained by the above-stated screening method are selected among the afore-mentioned test compounds, besides being the compound to promotes or inhibits activities of the promoter against the DNA of the present invention.

The compound thus obtained by the screening method may form its salts and as the salts of the compound, the salts with physiologically acceptable acids (e.g., inorganic acids) and bases (e.g., organic acids) are employed; among them, however, it is preferable to use the salts with physiologically acceptable acids addition salts above all. Examples of such salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The compound or its salts that promote the activities of the promoter against the DNA of the present invention can either promote expression of the polypeptide of the present invention, and promote functions of the polypeptide; therefore, they are useful as the prophylactic/therapeutic agents with safe and low toxic properties, for example, against diseases such as cancer, immunological diseases, respiratory tract diseases, digestive tract diseases, cardiovascular diseases, endocrinological diseases, infections, nervous system diseases and psychological diseases, etc.

Furthermore, the compounds derivatized from the compounds obtained by the above-stated screening can be also employed in the similar manner.

The drugs containing the compound or its salts that have been obtained by the screening method can be manufactured in the similar manners as those drugs containing the polypeptide of the present invention and its salts as stated above.

Since the thus obtained pharmaceutical preparation is safe and low toxic, the preparation can be administered to humans or mammals (e.g., rats, mice, guinea pigs, rabbits, sheep, swine, bovine, horses, cats, dogs, monkeys, etc.).

The doses of the compounds or their salts of the present invention vary depending on target diseases, subjects to be administered, and routes for administration, etc.; when a compound is orally administered for the purpose of treatment of diseases , for example, cancer, immunological diseases, respiratory tract diseases, digestive tract diseases, cardiovascular diseases, endocrinological diseases, infections, nervous system diseases and psychological diseases, etc., the dose to an adult (as 60 kg body weight) is normally about 0.1mg to about 100 mg, preferably about 1.0 mg to about 50 mg, more preferably about 1.0 mg to about 20 mg per day. In parenteral administration, the single dose of the said compound varies depending on subjects to be administered, target diseases, etc. but it is advantageous, e.g., for treatment of inflammatory diseases of the adult patient (as 60 kg body weight), to inject intravenously the compound to promote the promoter activity against the DNA of the present invention in a daily dose of about 0.01 mg to 30 mg, preferably about 0.1 mg to about 20 mg, more preferably about 0.1 mg to about 10 mg. For other animal species, the corresponding dose as converted per 60 kg body weight can be administered.

On the other hand, when the compound is orally administered to inhibit the promoter activity against the DNA of the present investigation, the dose of the compound to an adult (as 60 kg body weight) is normally about 0.1mg to about 100 mg, preferably about 1.0 mg to about 50 mg, more preferably about 1.0 mg to about 20 mg per day. In parenteral administration, the single dose of the said compound varies depending on subjects to be administered, target diseases, etc. but it is advantageous to administer in the parenteral form the compound to inhibit the promoter activity against the DNA of the present invention to the adult patient (as 60 kg body weight) in a daily dose of about 0.01 mg to 30 mg, preferably about 0.1 mg to about 20 mg, more preferably about 0.1 mg to about 10 mg. For other animal species, the corresponding dose as converted per 60 kg body weight can be administered.

Thus, the non-human mammals with the inadequately expressing DNA of the present invention are extremely useful in screening the compound or its salts that possess promoting or inhibiting activities of the promoter against the DNA of the present invention; therefore, they may greatly contribute to investigation of pathology of various diseases attributable to inadequate expression of the DNA of the present invention and development of the prophylactic/therapeutic agents.

In the specification and drawings, the codes of bases and amino acids are denoted in accordance with the IUPAC-IUB Commission on Biochemical Nomenclature or by the common codes in the art, and examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA :: ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : doxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagines
- Gln: : glutamine
- pGlu: : pyroglutamic acid

The substituents, protective groups and reagents, which are frequently used throughout the specification are shown by the following abbreviations.
- Me: : methyl
- Et: : ethyl
- Bu: : butyl
- Ph: : phenyl
- TC: : thiazolidine-4 (R)-carboxamide
- Tos: : p-toluenesulfonyl
- CHO: : formyl
- Bzl: : benzyl
- Cl₂Bz1: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: : jinitrophenol
- Trt: : trityl
- Bum: : t-butoxymethyl
- Fmoc: : N-9-fluorenylmethyoxycarbonyl
- HOBt: : 1-hydroxybenztriazole
- HOOBt: : 3,4-dihydro-3-hydroxy-4-oxo-1,2,3-benzotriazine
- HONB: : 1-hydroxy-5-norbornene-2,3-dicarboximide
- DCC: : N,N'-dicyclohexylcarbodiimide

The sequence idenfication numbers in the sequence listing of the specification indicates the following sequence, respectively.

### [SEQ ID NO:1]

This shows the amino acid sequence of human-derived polypeptide TGC-480 of the present invention.

### [SEQ ID NO:2]

This shows the amino acid sequence of human-derived polypeptide TGC-546 of the present invention.

### [SEQ ID NO:3]

This shows the amino acid sequence of human-derived polypeptide TGC-595 of the present invention.

### [SEQ ID NO:4]

This shows the amino acid sequence of human-derived polypeptide TGC-623 of the present invention.

### [SEQ ID NO:5]

This shows the amino acid sequence of human-derived polypeptide TGC-624 of the present invention.

### [SEQ ID NO:6]

This shows the amino acid sequence of human-derived polypeptide TGC-625 of the present invention.

### [SEQ ID NO:7]

This shows the amino acid sequence of human-derived polypeptide TGC-628 of the present invention.

### [SEQ ID NO:8]

This shows the amino acid sequence of human-derived polypeptide TGC-708 of the present invention.

### [SEQ ID NO:9]

This shows the amino acid sequence of human-derived polypeptide TGC-711 of the present invention.

### [SEQ ID NO:10]

This shows the amino acid sequence of human-derived polypeptide TGC-714 of the present invention.

### [SEQ ID NO:11]

This shows the amino acid sequence of human-derived polypeptide TGC-715 of the present invention.

### [SEQ ID NO:12]

This shows the amino acid sequence of human-derived polypeptide TGC-749 of the present invention.

### [SEQ ID NO:13]

This shows the amino acid sequence of human-derived polypeptide TGC-768 of the present invention.

### [SEQ ID NO:14]

This shows the amino acid sequence of human-derived polypeptide TGC-772 of the present invention.

### [SEQ ID NO:15]

This shows the amino acid sequence of human-derived polypeptide TGC-790 of the present invention.

### [SEQ ID NO:16]

This shows the base sequence of DNA encoding human-derived polypeptide TGC-480 of the present invention, which has the amino acid sequence shown by SEQ ID NO:1.

### [SEQ ID NO:17]

This shows the base sequence of DNA encoding human-derived polypeptide TGC-546 of the present invention, which has the amino acid sequence shown by SEQ ID NO:2.

### [SEQ ID NO:18]

This shows the base sequence of DNA encoding human-derived polypeptide TGC-595 of the present invention, which has the amino acid sequence shown by SEQ ID NO:3.

### [SEQ ID NO:19]

This shows the base sequence of DNA encoding human-derived polypeptide TGC-623 of the present invention, which has the amino acid sequence shown by SEQ ID NO:4.

### [SEQ ID NO:20]

This shows the base sequence of DNA encoding human-derived polypeptide TGC-624 of the present invention, which has the amino acid sequence shown by SEQ ID NO:5.

### [SEQ ID NO:21]

This shows the base sequence of DNA encoding human-derived polypeptide TGC-625 of the present invention, which has the amino acid sequence shown by SEQ ED NO:6.

### [SEQ ID NO:22]

This shows the base sequence of DNA encoding human-derived polypeptide TGC-628 of the present invention, which has the amino acid sequence shown by SEQ ID NO:7.

### [SEQ ID NO:23]

This shows the base sequence of DNA encoding human-derived polypeptide TGC-708 of the present invention, which has the amino acid sequence shown by SEQ ID NO:8.

### [SEQ ID NO:24]

This shows the base sequence of DNA encoding human-derived polypeptide TGC-711 of the present invention, which has the amino acid sequence shown by SEQ ID NO:9.

### [SEQ ID NO:25]

This shows the base sequence of DNA encoding human-derived polypeptide TGC-714 of the present invention, which has the amino acid sequence shown by SEQ ID NO: 10.

### [SEQ ID NO:26]

This shows the base sequence of DNA encoding human-derived polypeptide TGC-715 of the present invention, which has the amino acid sequence shown by SEQ ID NO: 11.

### [SEQ ID NO:27]

This shows the base sequence of DNA encoding human-derived polypeptide TGC-749 of the present invention, which has the amino acid sequence shown by SEQ ID NO:12.

### [SEQ ID NO:28]

This shows the base sequence of DNA encoding human-derived polypeptide TGC-768 of the present invention, which has the amino acid sequence shown by SEQ ID NO:13.

### [SEQ ID NO:29]

This shows the base sequence of DNA encoding human-derived polypeptide TGC-772 of the present invention, which has the amino acid sequence shown by SEQ ID NO:14.

### [SEQ ID NO:30]

This shows the base sequence of DNA encoding human-derived polypeptide TGC-790 of the present invention, which has the amino acid sequence shown by SEQ ID NO:15.

### [SEQ ID NO:31]

This shows the base sequence of the primer for 5' end as described in EXAMPLE 1.

### [SEQ ID NO:32]

This shows the base sequence of the primer for 3' end as described in EXAMPLE 1.

### [SEQ ED NO:33]

This shows the base sequence of the primer for 5' end as described in EXAMPLE 2.

### [SEQ ID NO:34]

This shows the base sequence of the primer for 3' end as described in EXAMPLE 2.

### [SEQ ID NO:35]

This shows the base sequence of the primer for 5' end as described in EXAMPLE 3.

### [SEQ ID NO:36]

This shows the base sequence of the primer for 3' end as described in EXAMPLE 3.

### [SEQ ID NO:37]

This shows the base sequence of the primer for 5' end as described in EXAMPLE 4.

### [SEQ ID NO:38]

This shows the base sequence of the primer for 3' end as described in EXAMPLE 4.

### [SEQ ID NO:39]

This shows the base sequence of the primer for 5' end as described in EXAMPLE 5.

### [SEQ ID NO:40]

This shows the base sequence of the primer for 3' end as described in EXAMPLE 5.

### [SEQ ID NO:41]

This shows the base sequence of the primer for 5' end as described in EXAMPLE 6.

### [SEQ ID NO:42]

This shows the base sequence of the primer for 3' end as described in EXAMPLE 6.

### [SEQ ID NO:43]

This shows the base sequence of the primer for 5' end as described in EXAMPLE 7.

### [SEQ ID NO:44]

This shows the base sequence of the primer for 3' end as described in EXAMPLE 7.

### [SEQ ID NO:45]

This shows the base sequence of the primer for 5' end as described in EXAMPLE 8.

### [SEQ ID NO:46]

This shows the base sequence of the primer for 3' end as described in EXAMPLE 8.

### [SEQ ID NO:47]

This shows the base sequence of the primer for 5' end as described in EXAMPLE 9.

### [SEQ ID NO:48]

This shows the base sequence of the primer for 3' end as described in EXAMPLE 9.

### [SEQ ID NO:49]

This shows the base sequence of the primer for 5' end as described in EXAMPLE 10.

### [SEQ ID NO:50]

This shows the base sequence of the primer for 3' end as described in EXAMPLE 10.

### [SEQ ID NO:51]

This shows the base sequence of the primer for 5' end as described in EXAMPLE 11.

### [SEQ ID NO:52]

This shows the base sequence of the primer for 3' end as described in EXAMPLE 11.

### [SEQ ID NO:53]

This shows the base sequence of the primer for 5' end as described in EXAMPLE 12.

### [SEQ ID NO:54]

This shows the base sequence of the primer for 3' end as described in EXAMPLE 12.

### [SEQ ID NO:55]

This shows the base sequence of the primer for 5' end as described in EXAMPLE 13.

### [SEQ ID NO:56]

This shows the base sequence of the primer for 3' end as described in EXAMPLE 13.

### [SEQ ID NO:57]

This shows the base sequence of the primer for 5' end as described in EXAMPLE 14.

### [SEQ ID NO:58]

This shows the base sequence of the primer for 3' end as described in EXAMPLE 14.

### [SEQ ID NO:59]

This shows the base sequence of the primer for 5' end as described in EXAMPLE 15.

### [SEQ ID NO:60]

This shows the base sequence of the primer for 3' end as described in EXAMPLE 15.

### [SEQ ID NO:61]

This shows the base sequence of the primer as described in EXAMPLE 16.

### [SEQ ID NO:62]

This shows the base sequence of the primer as described in EXAMPLE 16.

### [SEQ ID NO:63]

This shows the base sequence of the primer as described in EXAMPLE 17.

### [SEQ ID NO:64]

This shows the base sequence of the primer as described in EXAMPLE 17.

### [SEQ ID NO:65]

This shows the base sequence of the primer as described in EXAMPLE 18.

### [SEQ ID NO:66]

This shows the base sequence of the primer as described in EXAMPLE 18.

### [SEQ ID NO:67]

This shows the base sequence of the primer as described in EXAMPLE 19.

### [SEQ ID NO:68]

This shows the base sequence of the primer as described in EXAMPLE 19.

### [SEQ ID NO:69]

This shows the base sequence of the primer as described in EXAMPLE 20.

### [SEQ ID NO:70]

This shows the base sequence of the primer as described in EXAMPLE 20.

The present invention is described in detail below with reference to REFERENCE EXAMPLES and EXAMPLES, but is not deemed to limit the scope of the present invention thereto. The gene manipulation procedures using Escherichia coli were performed according to the methods described in the Molecular cloning.

### EXAMPLE 1

### Selection of TGC-480 from the data base and analysis of the base sequence

Among the EST database supplied by Smithkline Beecham LTD. (SB), the clone supposed to code the signal sequence contributing to secretion and the processing region were selected. In more detail, the DNA sequences of the EST were translated to the corresponding amino acid sequences and then the clone having the cluster of the hydrophobic amino acids (Leu, Ile, Val, Ala, etc.) after Met and further carrying the reserved sequence (Arg-Arg, Lys-Arg, Lys-Lys) at the processing region within the same frame was selected. As the result, HGS:558273 was discovered as the EST clone that satisfied these conditions. However, taking into account the fact that there was a possibility for the EST sequence to have deletion, insertion and miscoding of the base sequence as well as the fact that the EST sequence is a partial sequence of cDNA, SB was kindly requested to send this clone as TGC-480 to us and the whole base sequence of the insertion DNA fragment in the plasmid was determined by using fluorescent DNA sequencer (ABI PRINSMTH 377, Perkin Elmer). The results indicated that the insertion sequence (cDNA) of the clone carried the open reading frame for 378 bases as represented by SEQ ID NO:16, which encoded the polypeptide comprising 125 amino acid residue as shown by SEQ ID NO:1. The residue consisting of 21 amino acids at N-terminus as shown by SEQ ID NO: 1 was anticipated to be the signal sequence for secretion. It was also verified that this clone expressed in brain, testis, heart, etc.

The cDNA fragment could be obtained by conventional methods, based on the above information. In other words, using the base sequence as described in SEQ ID NO:16, the primers for PCR corresponding to 5' end and 3' end (e.g., ATGGCCAAGTACCTGGCCCAGATCA and TCACGTATGGGGCATCTGCCCTTTT) were prepared, followed by employment of the cDNA library of the above-stated tissues (e.g., brain, testis, heart, etc.) or RT-PCR with use of mRNA derived from the aforementioned tissues as a template to obtain the cDNA fragment. In detail, the solution 50 µl containing 5 pmol of the each primer, 5 µl of 100 mM Tris-HCl buffer (pH9.0), 5 µl of 500 mM potassium chloride solution, 3 µl of 25 mM magnesium chloride solution, 4 µl of 2.5 mM deoxyribonucleotide solution, 1 µl of cDNA solution, and 0.5 µl of TaKaRa Taq™ was prepared. The PCR reaction was performed according to the follwing program, namely, the resultant solution was placed at 95°C for 1 minute in TaKaRa PCR Thermal Cycler MP (Takara shuzo Co., Ltd.), then at 95°C for 30 seconds, at 65°C for 1 minute and at 72°C for 2 minutes; this cycle was repeated 35 times in total and further reacted at 72°C for 10 minutes to obtain the target PCR fragment.

### EXAMPLE 2

### Selection of TGC-546 from the data base and analysis of the base sequence

After selection of HGS:447917 by the similar method as described in EXAMPLE 1, this clone was received as TGC-546 and then the base sequence was confirmed. As the result, it was revealed that the insertion sequence (cDNA) of the clone carried the open reading frame for 366 bases as represented by SEQ ID NO: 17, which encoded the polypeptide comprising 121 amino acid residue as represented by SEQ ID NO:2. The residue consisting of 23 amino acids at N-terminus of the amino acid sequence as represented by SEQ ID NO:2 was anticipated to be the signal sequence for secretion. It was also verified that this clone expressed in epididymis.

The cDNA fragment could be obtained by conventional methods, based on the above information. In other words, using the base sequence as described in SEQ ID NO:17, the primers for PCR corresponding to 5' end and 3' end (e.g., ATGCACAGATCAGAGCCATTTCTGA and TTACAGTAGTGGCAGTAACACTTGG) were prepared, followed by employment of the cDNA library of the above-stated tissues (e.g., dpididymis, etc.) or RT-PCR with use of mRNA derived from the aforementioned tissues as a template to obtain the cDNA fragment. The reaction conditions to be applied to PCR were those as described in EXAMPLE 1.

### EXAMPLE 3

### Selection of TGC-595 from the data base and analysis of the base sequence

After selection of HGS: 1006634 by the similar method as described in EXAMPLE 1, this clone was received as TGC-595 and then the base sequence was confirmed. As the result, it was revealed that the insertion sequence (cDNA) of the clone carried the open reading frame for 672 bases as referred to as SEQ ID NO:18, which encoded the polypeptide comprising 223 amino acid residue as shown by SEQ ID NO:3. The residue consisting of 19 amino acids at N-terminus of the amino acid sequence as shown by SEQ ID NO:3 was anticipated to be the signal sequence for secretion. It was also verified that this clone expressed in spinal cord, T cells, retina, etc.

The cDNA fragment could be obtained by conventional methods, based on the above information. In other words, using the base sequence as described in SEQ ID NO:18, the primers for PCR corresponding to 5' end and 3' end (e.g., ATGAAGTTCGTCCCCTGCCTCCTGC and TCACCCTCGGAAGAAGCTGATGAGA) were prepared, followed by employment of the cDNA library of the above-stated tissues (e.g., spinal cord, T cells, retina, etc.) or RT-PCR with use of mRNA derived from the aforementioned tissues as a template to obtain the cDNA fragment. The reaction conditions to be applied to PCR were those as described in EXAMPLE 1.

### EXAMPLE 4

### Selection of TGC-623 from the data base and analysis of the base sequence

After selection of HGS:92551 by the similar method as described in EXAMPLE 1, this clone was received as TGC-623 and then the base sequence was confirmed. As the result, it was revealed that the insertion sequence (cDNA) of the clone carried the open reading frame for 747 bases as referred to as SEQ ID NO: 19, which encoded the polypeptide comprising 248 amino acid residue as shown by SEQ ID NO:4. The residue consisting of 21 amino acids at N-terminus of the amino acid sequence as shown by SEQ ID NO:4 was anticipated to be the signal sequence for secretion. It was also verified that this clone expressed in cerebellum, adrenal, etc.

The cDNA fragment could be obtained by conventional methods, based on the above information. In other words, using the base sequence as described in SEQ ID NO:19, the primers for PCR corresponding to 5' end and 3' end (e.g., ATGGGACCTGTGCGGTTGGGAATAT and TCAAAGATCTTCTCGGTCAAGTTTG) were prepared, followed by employment of the cDNA library of the above-stated tissues (e.g., cerebellum, adrenal, etc.) or RT-PCR with use of mRNA derived from the aforementioned tissues as a template to obtain the cDNA fragment. The reaction conditions to be applied to PCR were those as described in EXAMPLE 1.

### EXAMPLE 5

### Selection of TGC-624 from the data base and analysis of the base sequence

After selection of HGS:1731120 by the similar method as described in EXAMPLE 1, this clone was received as TGC-624 and then the base sequence was confirmed. As the result, it was revealed that the insertion sequence (cDNA) of the clone carried the open reading frame for 522 bases as referred to as SEQ ID NO:20, which encoded the polypeptide comprising 173 amino acid residue as shown by SEQ ID NO:5. The residue consisting of 19 amino acids at N-terminus of the amino acid sequence as shown by SEQ ID NO:5 was anticipated to be the signal sequence for secretion. It was also verified that this clone expressed in dendritic cells, T cells, etc.

The cDNA fragment could be obtained by conventional methods, based on the above information. In other words, using the base sequence as described in SEQ ID NO:20, the primers for PCR corresponding to 5' end and 3' end (e.g., ATGTTTTGCCCACTGAAACTCATCC and TCATGAAAATATCCATTCTACCTTG) were prepared, followed by employment of the cDNA library of the above-stated tissues (e.g., dendritic cells, T cells, etc.) or RT-PCR with use of mRNA derived from the aforementioned tissues as a template to obtain the cDNA fragment. The reaction conditions to be applied to PCR were those as described in EXAMPLE 1.

### EXAMPLE 6

### Selection of TGC-625 from the data base and analysis of the base sequence

After selection of HGS:1014817 by the similar method as described in EXAMPLE 1, this clone was received as TGC-625 and then the base sequence was confirmed. As the result, it was revealed that the insertion sequence (cDNA) of the clone carried the open reading frame for 786 bases as referred to as SEQ ID NO:21, which encoded the polypeptide comprising 261 amino acid residue as shown by SEQ ID NO:6. The residue consisting of 20 amino acids at N-terminus of the amino acid sequence as shown by SEQ ID NO:6 was anticipated to be the signal sequence for secretion. It was also verified that this clone expressed in vascular endothelial cells, bone marrow, etc.

The cDNA fragment could be obtained by conventional methods, based on the above information. In other words, using the base sequence as described in SEQ ID NO:21, the primers for PCR corresponding to 5' end and 3' end (e.g., ATGGAACTGCTTCAAGTGACCATTC and TCAGTTCTTGGTTTTTCCTTGTGCA) were prepared, followed by employment of the cDNA library of the above-stated tissues (e.g., vascular endothelial cells, bone marrow, etc.) or RT-PCR with use of mRNA derived from the aforementioned tissues as a template to obtain the cDNA fragment. The reaction conditions to be applied to PCR were those as described in EXAMPLE 1.

### EXAMPLE 7

### Selection of TGC-628 from the data base and analysis of the base sequence

After selection of HGS: 1878022 by the similar method as described in EXAMPLE 1, this clone was received as TGC-628 and then the base sequence was confirmed. As the result, it was revealed that the insertion sequence (cDNA) of the clone carried the open reading frame for 732 bases as referred to as SEQ ID NO:22, which encoded the polypeptide comprising 243 amino acid residue as shown by SEQ ID NO:7. The residue consisting of 30 amino acids at N-terminus of the amino acid sequence as shown by SEQ ID NO:7 was anticipated to be the signal sequence for secretion. It was also verified that this clone expressed in thymus, placenta, etc.

The cDNA fragment could be obtained by conventional methods, based on the above information. In other words, using the base sequence as described in SEQ ID NO:22, the primers for PCR corresponding to 5' end and 3' end (e.g., ATGCGACCCCAGGGCCCCGCCGCCT and TTATTTTGGTAGTTCTTCAATAATG) were prepared, followed by employment of the cDNA library of the above-stated tissues (e.g., thymus, placenta, etc.) or RT-PCR with use of mRNA derived from the aforementioned tissues as a template to obtain the cDNA fragment. The reaction conditions to be applied to PCR were those as described in EXAMPLE 1.

### EXAMPLE 8

### Selection of TGC-708 from the data base and analysis of the base sequence

After selection of HGS:2346555 by the similar method as described in EXAMPLE 1, this clone was received as TGC-708 and then the base sequence was confirmed. As the result, it was revealed that the insertion sequence (cDNA) of the clone carried the open reading frame for 450 bases as referred to as SEQ ID NO:23, which encoded the polypeptide comprising 149 amino acid residue as shown by SEQ ID NO:8. The residue consisting of 18 amino acids at N-terminus of the amino acid sequence as shown by SEQ ID NO:8 was anticipated to be the signal sequence for secretion. It was also verified that this clone expressed in monocytes.

The cDNA fragment could be obtained by conventional methods, based on the above information. In other words, using the base sequence as described in SEQ ID NO:23, the primers for PCR corresponding to 5' end and 3' end (e.g., ATGAAGTTACAGTGTGTTTCCCTTT and TCAGGAGGCCGATGGGGGCCAGCAC) were prepared, followed by employment of the cDNA library of the above-stated tissues (e.g., monocytes, etc.) or RT-PCR with use of mRNA derived from the aforementioned tissues as a template to obtain the cDNA fragment. The reaction conditions to be applied to PCR were those as described in EXAMPLE 1.

### EXAMPLE 9

### Selection of TGC-711 from the data base and analysis of the base sequence

After selection of HGS:809616 by the similar method as described in EXAMPLE 1, this clone was received as TGC-711 and then the base sequence was confirmed. As the result, it was revealed that the insertion sequence (cDNA) of the clone carried the open reading frame for 411 bases as referred to as SEQ ID NO:24, which encoded the polypeptide comprising 136 amino acid residue as shown by SEQ ID NO:9. The residue consisting of 20 amino acids at N-tenninus of the amino acid sequence as shown by SEQ ID NO:9 was anticipated to be the signal sequence for secretion. It was also verified that this clone expressed in cerebellum, lung, etc.

The cDNA fragment could be obtained by conventional methods, based on the above information. In other words, using the base sequence as described in SEQ ID NO:24, the primers for PCR corresponding to 5' end and 3' end (e.g., ATGGCCAGCCTGGGGCTGCTGCTCC and TCATGAGGCTCCTGCAGAGGTCTGA) were prepared, followed by employment of the cDNA library of the above-stated tissues (e.g., cerebellum, lung, etc.) or RT-PCR with use of mRNA derived from the aforementioned tissues as a template to obtain the cDNA fragment. The reaction conditions to be applied to PCR were those as described in EXAMPLE 1.

### EXAMPLE 10

### Selection of TGC-714 from the data base and analysis of the base sequence

After selection of HGS: 1260352 by the similar method as described in EXAMPLE 1, this clone was received as TGC-714 and then the base sequence was confirmed. As the result, it was revealed that the insertion sequence (cDNA) of the clone carried the open reading frame for 372 bases as referred to as SEQ ID NO:25, which encoded the polypeptide comprising 123 amino acid residue as shown by SEQ ID NO:10. The residue consisting of 22 amino acids at N-terminus of the amino acid sequence as shown by SEQ ID NO:10 was anticipated to be the signal sequence for secretion. It was also verified that this clone expressed in epididymis.

The cDNA fragment could be obtained by conventional methods, based on the above information. In other words, using the base sequence as described in SEQ ID NO:25, the primers for PCR corresponding to 5' end and 3' end (e.g., ATGAAACTCCTGCTGCTGGCTCTTC and TCATGAGCTATGGTGAACATTTGGA) were prepared, followed by employment of the cDNA library of the above-stated tissues (e.g., epididymis, etc.) or RT-PCR with use of mRNA derived from the aforementioned tissues as a template to obtain the cDNA fragment. The reaction conditions to be applied to PCR were those as described in EXAMPLE 1.

### EXAMPLE 11

### Selection of TGC-715 from the data base and analysis of the base sequence

After selection of HGS:81772 by the similar method as described in EXAMPLE 1, this clone was received as TGC-715 and then the base sequence was confirmed. As the result, it was revealed that the insertion sequence (cDNA) of the clone carried the open reading frame for 492 bases as referred to as SEQ ID NO:26, which encoded the polypeptide comprising 163 amino acid residue as shown by SEQ ID NO:11. The residue consisting of 20 amino acids at N-terminus of the amino acid sequence as shown by SEQ ID NO:11 was anticipated to be the signal sequence for secretion. It was also verified that this clone expressed in epididymis.

The cDNA fragment could be obtained by conventional methods, based on the above information. In other words, using the base sequence as described in SEQ ID NO:26, the primers for PCR corresponding to 5' end and 3' end (e.g., ATGGGCGGCCTGCTGCTGGCTGCTT and CTACTGTGACAGGAAGCCCAGGCTC) were prepared, followed by employment of the cDNA library of the above-stated tissues (e.g., epididymis, etc.) or RT-PCR with use of mRNA derived from the aforementioned tissues as a template to obtain the cDNA fragment. The reaction conditions to be applied to PCR were those as described in EXAMPLE 1.

### EXAMPLE 12

### Selection of TGC-749 from the data base and analysis of the base sequence

After selection of HGS:1379897 by the similar method as described in EXAMPLE 1, this clone was received as TGC-749 and then the base sequence was confirmed. As the result, it was revealed that the insertion sequence (cDNA) of the clone carried the open reading frame for 906 bases as referred to as SEQ ID NO:27, which encoded the polypeptide comprising 301 amino acid residue as shown by SEQ ID NO:12. The residue consisting of 18 amino acids at N-terminus of the amino acid sequence as shown by SEQ ID NO:12 was anticipated to be the signal sequence for secretion. It was also verified that this clone expressed in T cells, placenta, liver, large intestine, etc.

The cDNA fragment could be obtained by conventional methods, based on the above information. In other words, using the base sequence as described in SEQ ID NO:27, the primers for PCR corresponding to 5' end and 3' end (e.g., ATGGCCCGGCATGGGTTACCGCTGC and TTACAGCTCCCCTGGCGGCCGGCCT) were prepared, followed by employment of the cDNA library of the above-stated tissues (e.g., T cells, placenta, liver, large intestine, etc.) or RT-PCR with use of mRNA derived from the aforementioned tissues as a template to obtain the cDNA fragment. The reaction conditions to be applied to PCR were those as described in EXAMPLE 1.

### EXAMPLE 13

### Selection of TGC-768 from the data base and analysis of the base sequence

After selection of HGS:398232 by the similar method as described in EXAMPLE 1, this clone was received as TGC-768 and then the base sequence was confirmed. As the result, it was revealed that the insertion sequence (cDNA) of the clone carried the open reading frame for 210 bases as referred to as SEQ ID NO:28, which encoded the polypeptide comprising 69 amino acid residue as shown by SEQ ID NO:13. The residue consisting of 26 amino acids at N-terminus of the amino acid sequence as shown by SEQ ID NO:13 was anticipated to be the signal sequence for secretion. It was also verified that this clone expressed in testis.

The cDNA fragment could be obtained by conventional methods, based on the above information. In other words, using the base sequence as described in SEQ ID NO:28, the primers for PCR corresponding to 5' end and 3' end (e.g., ATGTGCTGGCTGCGGGCATGGGGCC and TTATCTATTCATCATATATTTCTTA) were prepared, followed by employment of the cDNA library of the above-stated tissues (e.g., testis, etc.) or RT-PCR with use of mRNA derived from the aforementioned tissues as a template to obtain the cDNA fragment. The reaction conditions to be applied to PCR were those as described in EXAMPLE 1.

### EXAMPLE 14

### Selection of TGC-772 from the data base and analysis of the base sequence

After selection of HGS:2079036 by the similar method as described in EXAMPLE 1, this clone was received as TGC-772 and then the base sequence was confirmed. As the result, it was revealed that the insertion sequence (cDNA) of the clone carried the open reading frame for 210 bases as referred to as SEQ ID NO:29, which encoded the polypeptide comprising 69 amino acid residue as shown by SEQ ID NO:14. The residue consisting of 23 amino acids at N-terminus of the amino acid sequence as shown by SEQ ID NO:14 was anticipated to be the signal sequence for secretion. It was also verified that this clone expressed in pancreas, placenta, etc.

The cDNA fragment could be obtained by conventional methods, based on the above information. In other words, using the base sequence as described in SEQ ID NO:29, the primers for PCR corresponding to 5' end and 3' end (e.g., ATGGGGTTCCCGGCCGCGGCGCTGC and CTACGCCGAGACCGTGGGCCTGCGG) were prepared, followed by employment of the cDNA library of the above-stated tissues (e.g., pancreas, placenta, etc.) or RT-PCR with use of mRNA derived from the aforementioned tissues as a template to obtain the cDNA fragment. The reaction conditions to be applied to PCR were those as described in EXAMPLE 1.

### EXAMPLE 15

### Selection of TGC-790 from the data base and analysis of the base sequence

After selection of HGS:2450362 by the similar method as described in EXAMPLE 1, this clone was received as TGC-790 and then the base sequence was confirmed. As the result, it was revealed that the insertion sequence (cDNA) of the clone carried the open reading frame for 594 bases as referred to as SEQ ID NO:30, which encoded the polypeptide comprising 197 amino acid residue as shown by SEQ ID NO:15. The residue consisting of 26 amino acids at N-terminus of the amino acid sequence as shown by SEQ ID NO:15 was anticipated to be the signal sequence for secretion. It was also verified that this clone expressed in placenta, etc.

The cDNA fragment could be obtained by conventional methods, based on the above information. In other words, using the base sequence as described in SEQ ID NO:30, the primers for PCR corresponding to 5' end and 3' end (e.g., ATGCGAGGTGGCAAATGCAACATGC and TCATAAACTTGTGTTGGGCTTTAGG) were prepared, followed by employment of the cDNA library of the above-stated tissues (e.g., placenta, etc.) or RT-PCR with use of mRNA derived from the aforementioned tissues as a template to obtain the cDNA fragment. The reaction conditions to be applied to PCR were those as described in EXAMPLE 1.

### EXAMPLE 16

### Secretion expression of TGC-480 product in COS7 cells

The expression vector to express TGC-480 product in animal cells was obtained by insertion of the DNA fragment containing ORF encoding the TGC-480 product into the expression vector pCAN618 for animal cells.

First of all, the PCR was performed by using the synthetic DNA [5'-ACGCTCGAGTTACTTGTCATCGTCGTCCTTGTAGTCCGTATGGGGCATCT GCCCTTTTTC-3' : (SEQ ID NO:62)], which was designed to locate the synthetic DNA [5'-TCGGAATTCGCCATGGCCAAGTACCTGGCCCAGATC-3' : (SEQ ID NO:61)] having the recognition site for the restriction enzyme Eco RI immediately before the translation start codon initiator, the FLAG sequence consisting of 8 amino acids (Asp-Tyr-Lys-Asp-Asp-Asp-Asp-Lys) at C-terminus of the TGC-480 protein, and subsequent stop codon and the recognition site for the restriction enzyme Xho I, by using the cDNA fragment encoding the TGC-480 protein obtained in EXAMPLE 1 as a template. The PCR reaction was performed according the following program, with use of Pyrobest DNA Polymerase (Takara Shuzo Co., Ltd.); namely, the reaction mixture was placed at 94°C for 1 minute, then at 98°C for 10 seconds, at 60°C for 30 seconds and at 72°C for 1 minute; this cycle was repeated 25 times in total. Finally, extension reaction at 72°C for 10 minutes was performed to obtain the DNA fragment containing the ORF of TGC-480. The resultant DNA fragment was cleaved with the restriction enzyme Eco RI and Xho I, followed by insertion of them into Eco RI/Xho I sites in pCAN618 to obtain the expression vector for TGC-480 protein, pCAN618/TGC-480FLAG for animal cells.

In the DMEM (medium; Gibco BRL) containing 10% FBS (bovine fetal serum), 4x10⁵ COS7 cells were incubated in a 6-well plate for 24 hours. The expression vector pCAN618/TGC480FLAG DNA was introduced into these cells with use of LipofectAMINE (Gibco BRL) and then cultured for further 18 hours. The medium was replaced with Opti-MEM (Medium ; Gibco BRL) containing 0.05% CHAPS and cultured for further 24 hours, followed by recovery of the supernatant. The supernatant was subjected to centrifugation to remove the floating cells, and then condensed to 1/10 by ultrafiltration (Centricon; Amicon). The resultant solution was added with the same volume of SDS-Sample Buffer containing 2-mercaptoethanol, followed by electrophoresis in 10 - 25 % SDS-PAGE (TEFCO). After transferring this to the PVDF membrane (Amersham), Western Blot analysis was performed. Anti-FLAG mouse IgG (10µg/ml ; Sigma) was used as the primary antibody while HRP (Horseraddish peroxidase)-labeled anti-mouse IgG antibody (x 2000 dilution; Amersham) was used as the secondary antibody, with use of ECLplus Western Blot Detection System (Amershiam) for detection. As the result, it was revealed that TGC-480 protein was secreted into the culture supernatant (Fig. 1).

### EXAMPLE 17

### Secretion expression of TGC-623 product in COS7 cells

The expression vector to express TGC-623 product in animal cells was obtained by insertion of the DNA fragment containing ORF encoding the TGC-623 product into the expression vector pCAN618FLAG for animal cells. pCAN618FLAg was derived from the plasmid vector pCAN618, and pCAN618FLAG can express the target protein as the FLAG fused protein, by coinciding the reading frame of the base sequence encoding the FLAG sequence for 8 amino acids existing immediately after Sal I site (Asp-Tyr-Lys-Asp-Asp-Asp-Asp-Lys) to the end codon.

First of all, the PCR was performed by using the synthetic DNA [5'-AGGCAAGTCGACAAGATCTTCTCGGTCAAGTTTGGGGTGGCTTCCTGTC TTGGTCAT-3': (SEQ ID NO:68)], which was designed to locate the restriction enzyme Sal I recognition site at C end of TGC-623 protein, and the synthetic DNA [5'-TAGACGAATTCCCACCATGGGACCTGTGCGGTTGGGAATATTGC-3' : (SEQ ID NO:67)] having the recognition site for the restriction enzyme Eco RI immediately before the translation start codon initiator by using the cDNA fragment encoding TGC-623 protein obtained in EXAMPLE 4. The PCR reaction was performed according to the following program, with use of Pyrobest DNA Polymerase (Takara); namely, the reaction mixture was placed at 94°C for 1 minute, then at 98°C for 10 seconds, at 57°C for 30 seconds and at 72°C for 1 minute; this cycle was repeated 25 times in total. Finally, extension reaction at 72°C for 10 minutes was performed to obtain the DNA fragment containing the ORF of TGC-623. The resultant DNA fragment was cleaved with the restriction enzyme Eco RI and Sal I, followed by insertion of them into Eco RI/Sal I sites in pCAN618FLAG to obtain the expression vector for TGC-623 protein, or pCAN618/TGC-623FLAG for animal cells. This expression vector was introduced into COS7 cells in the similar manners for those in EXAMPLE 16, and the culture supernatant was prepared, thereby performing the Western Blot analysis. As the result, it was revealed that TGC-623 protein was secreted into the culture supernatant.(Fig. 1)

### EXAMPLE 18

### Secretion expression of TGC-711 product in COS7 cells

The expression vector to express TGC-711 product in animal cells was obtained in the similar manner as those for TGC-480 product described in EXAMPLE 16.

First of all, the PCR was performed by using the synthetic DNA [5'-ACGCTCGAGTTACTTGTCATCGTCGTCCTTGTAGTCTGAGGCTCCTGCAG AGGTCTGAGA-3': (SEQ ID NO:64)], which was designed to locate the FLAG sequence (Asp-Tyr-Lys-Asp-Asp-Asp-Asp-Lys) and subsequently the end codon as well as the recognition site for the restriction enzyme Xho I in this sequence order, and the synthetic DNA [5'-TCGGAATTCGCCATGGCCAGCCTGGGGCTGCTGCTC-3': (SEQ ID NO:63)] having as a template the recognition site for the restriction enzyme Eco RI immediately before the translation start codon initiator, by using the cDNA fragment encoding TGC-711 protein obtained in EXAMPLE 9 as a template. The PCR reaction and subsequent treatments were performed under the similar conditions as those in EXAMPLE 16, to obtain the expression vector for human TGC-711 protein, or pCAN618/TGC-711FLAG for animal cells. This expression vector was introduced into COS7 cells in the similar manners for those in EXAMPLE 16, and the culture supernatant was prepared, thereby being used for performing the Western Blot analysis. As the result, it was revealed that TGC-711 protein was secreted into the culture supernatant (Fig. 1).

### EXAMPLE 19

### Secretion expression of TGC-714 product in COS7 cells

The expression vector to express TGC-714 product in animal cells was obtained in the similar manner as those for TGC-480 product described in EXAMPLE 16.

First of all, the PCR was performed by using the synthetic DNA [5'-ACGCTCGAGTTACTTGTCATCGTCGTCCTTGTAGTCTGAGCTATGGTGAA CATTTGGAAG-3': (SEQ ID NO:66)], which was designed to locate the FLAG sequence (Asp-Tyr-Lys-Asp-Asp-Asp-Asp-Lys) and subsequently the end codon as well as the recognition site for the restriction enzyme Xho I in this sequence order, and the synthetic DNA [5'-TCGGAATTCACCATGAAACTCCTGCTGCTGGCTCTT-3' : (SEQ ID NO:65)] having the recognition site for the restriction enzyme Eco RI immediately before the translation start codon initiator, by using the cDNA fragment encoding TGC-711 protein obtained in EXAMPLE 9 as a template. The PCR reaction was performed according to the following program, with use of Pyrobest DNA Polymerase (Takara); namely, the reaction mixture was placed at 94°C for 1 minute, then at 98°C for 10 seconds, at 55°C for 30 seconds and at 72°C for 30 seconds; this cycle was repeated 25 times in total. Finally, extension reaction at 72°C for 10 minutes was performed to obtain the DNA fragment containing the ORF of TGC-714. The resultant DNA fragment was cleaved with the restriction enzyme Eco RI and Xho I, followed by insertion of them into Eco RI/Xho I sites in pCAN618 to obtain the expression vector for TGC-714 protein , or pCAN618/TGC-714FLAG for animal cells.

This expression vector was introduced into COS7 cells in the similar manners for those in EXAMPLE 16, and the culture supernatant was prepared, thereby being used for performing the Western Blot analysis. As the result, it was revealed that TGC-714 protein was secreted into the culture supernatant.(Fig. 1)

### EXAMPLE 20

### Secretion expression of TGC-715 product in COS7 cells

The expression vector to express TGC-715 product in animal cells was obtained in the similar manner as those for TGC-623 product described in EXAMPLE 17.

First of all, the PCR was performed by using the synthetic DNA [5'-CTGGGCGTCGACCTGTGACAGGAAGCCCAGGCTCCTGCTCCACT-3' : (SEQ ID NO:70)], which was designed to locate recognition site for the restriction enzyme, or Sal I at C end of the TGC-715 protein, and the synthetic DNA [5'-GTGTAGAATTCCCACCATGGGCGGCCTGCTGCTGGCTGCTTTTCTGGCTT T-3' : (SEQ ID NO:69)] having the recognition site for the restriction enzyme Eco RI immediately before the translation start codon initiator, by using the cDNA fragment encoding TGC-715 protein obtained in EXAMPLE 11 as a template. The PCR reaction was performed under the similar conditions as those in EXAMPLE 16, and the obtained DNA fragment was introduced into the Eco RI/Sal I sites of pCAN618FLAG by the similar methods for those in EXAMPLE 17 to obtain the expression vector for human TGC-715 protein, or pCAN618/TGC-715FLAG for animal cells. This expression vector was introduced into COS7 cells in the similar manners for those in EXAMPLE 16, and the culture supernatant was prepared, thereby being used for performing the Western Blot analysis. As the result, it was revealed that TGC-715 protein was secreted into the culture supernatant.(Fig. 1)

### INDUSTRIAL APPLICABILITY

The polypeptide of the present invention can be used as the tissue marker, cancer marker, molecular weight marker, pathological marker for the purpose of fundamental studies in the fields, for example, biology, physiology, pharmacology and medicine, as well as for detection of the binding protein to the polypeptide of the present invention. It is anticipated that the polypeptide of the present invention possesses more than one biological activity, for example, including anti-cancer activity, anti-inflammatory activity, hematopoietic activity, anti-coagulation activity, cell migration activity, cell growth stimulating activity, cell differentiation inducing activity, immuno-modulating activity, ligand activity, etc; therefore, the polypeptide of the present invention can be used for diagnosis, treatment and prophylaxis of the diseases due to such effects. Furthermore, the polypeptide of the present invention is useful as the reagent for screening of the compound or its salts that either promotes or inhibits activities of the polypeptide of the present invention. In addition, since the antibody against the polypeptide of the present invention can specifically recognize the polypeptide of the present invention, it can be employed for detection, quantification and neutralization of the polypeptide of the present invention in the test solution.

## Claims

1. A polypeptide comprising the same or substantially the same amino acid sequence as at least one amino acid sequence selected among SEQ ID NO: 1 or SEQ ID NO: 15, or a salt thereof.

2. A manufacturing method for the polypeptide or its salt according to claim 1, which comprises culture of the transformant that was transformed by the recombinant vector containing DNA encoding the polypeptide described in claim 1, and production of the polypeptide.

3. An antibody to the polypeptide or its salt according to claim 1.

4. A method of screening a compound or its salt that either promotes or inhibits activities of the polypeptide or its salts according to claim 1, which comprises using the polypeptide or its salt according to claim 1.

5. A kit for screening a compound or its salt that either promotes or inhibits activities of the polypeptide or its salts according to claim 1, which comprises the polypeptide or its salt according to claim 1.

6. A compound or its salt that either promotes or inhibits activities of the polypeptide or its salt according to claim 1, which can be obtained by the screening method according to claim 4 or the kit for screening according to claim 5.

7. A pharmaceutical comprising a compound or its salt that either promotes or inhibits activities of the polypeptide or its salts according to claim 1, which can be obtained by the screening method according to claim 4 or the kit for screening according to claim 5.

8. A pharmaceutical composition comprising the polypeptide or its salt according to claim 1.


SEQUENCE LISTINGS

<110> Takeda Chemical Industries, Ltd.

<120> Novel Polypeptide

<130> 2609WOOP

<150> JP 11-140229

<151> 1999-05-20

<160> 70

<210> 1

<211> 125

<212> PRT

<213> Human

<400> 1

Met Ala Lys Tyr Leu Ala Gln Ile Ile Val Met Gly Val Gln Val Val

                5                   10                  15

Gly Arg Ala Phe Ala Arg Ala Leu Arg Gln Glu Phe Ala Ala Ser Arg

            20                  25                  30

Ala Ala Ala Asp Ala Arg Gly Arg Ala Gly His Arg Ser Ala Ala Ala

        35                  40                  45

Ser Asn Leu Ser Gly Leu Ser Leu Gln Glu Ala Gln Gln Ile Leu Asn

    50                  55                  60

Val Ser Lys Leu Ser Pro Glu Glu Val Gln Lys Asn Tyr Glu His Leu

65                  70                  75                  80

Phe Lys Val Asn Asp Lys Ser Val Gly Gly Ser Phe Tyr Leu Gln Ser

                85                  90                  95

Lys Val Val Arg Ala Lys Glu Arg Leu Asp Glu Glu Leu Lys Ile Gln

            100                 105                 110

Ala Gln Glu Asp Arg Glu Lys Gly Gln Met Pro His Thr

        115                 120                 125

<210> 2

<211> 121

<212> PRT

<213> Human

<400> 2

Met His Arg Ser Glu Pro Phe Leu Lys Met Ser Leu Leu Ile Leu Leu

                5                   10                  15

Phe Leu Gly Leu Ala Glu Ala Cys Thr Pro Arg Glu Val Asn Leu Leu

            20                  25                  30

Lys Gly Ile Ile Gly Leu Met Ser Arg Leu Ser Pro Asp Glu Ile Leu

        35                  40                  45

Gly Leu Leu Ser Leu Gln Val Leu His Glu Glu Thr Ser Gly Cys Lys

    50                  55                  60

Glu Glu Val Lys Pro Phe Ser Gly Thr Thr Pro Ser Arg Lys Pro Leu

65                  70                  75                  80

Pro Lys Arg Lys Asn Thr Trp Asn Phe Leu Lys Cys Ala Tyr Met Val

                85                  90                  95

Met Thr Tyr Leu Phe Val Ser Tyr Asn Lys Gly Asp Trp Phe Thr Phe

            100                 105                 110

Ser Ser Gln Val Leu Leu Pro Leu Leu

        115                 120

<210> 3

<211> 223

<212> PRT

<213> Human

<400> 3

Met Lys Phe Val Pro Cys Leu Leu Leu Val Thr Leu Ser Cys Leu Gly

                5                   10                  15

Thr Leu Gly Gln Ala Pro Arg Gln Lys Gln Gly Ser Thr Gly Glu Glu
            20                  25                  30
Phe His Phe Gln Thr Gly Gly Arg Asp Ser Cys Thr Met Arg Pro Ser
        35                  40                  45
Ser Leu Gly Gln Gly Ala Gly Glu Val Trp Leu Arg Val Asp Cys Arg
    50                  55                  60
Asn Thr Asp Gln Thr Tyr Trp Cys Glu Tyr Arg Gly Gln Pro Ser Met
65                  70                  75                  80
Cys Gln Ala Phe Ala Ala Asp Pro Lys Ser Tyr Trp Asn Gln Ala Leu
                85                  90                  95
Gln Glu Leu Arg Arg Leu His His Ala Cys Gln Gly Ala Pro Val Leu
            100                 105                 110
Arg Pro Ser Val Cys Arg Glu Ala Gly Pro Gln Ala His Met Gln Gln
        115                 120                 125
Val Thr Ser Ser Leu Lys Gly Ser Pro Glu Pro Asn Gln Gln Pro Glu
    130                 135                 140
Ala Gly Thr Pro Ser Leu Ser Pro Lys Ala Thr Val Lys Leu Thr Gly
145                 150                 155                 160
Ala Thr Gln Leu Gly Lys Asp Ser Met Glu Glu Leu Gly Lys Ala Lys
                165                 170                 175
Pro Thr Thr Gly Pro Thr Ala Lys Pro Thr Gln Pro Gly Pro Arg Pro
            180                 185                 190
Gly Gly Asn Glu Glu Ala Lys Lys Lys Ala Trp Glu His Cys Trp Lys
        195                 200                 205
Pro Phe Gln Ala Leu Cys Ala Phe Leu Ile Ser Phe Phe Arg Gly
    210                 215                 220
<210> 4
<211> 248
<212> PRT

<213> Human

<400> 4

Met Gly Pro Val Arg Leu Gly Ile Leu Leu Phe Leu Phe Leu Ala Val

                5                   10                  15

His Glu Ala Trp Ala Gly Met Leu Lys Glu Glu Asp Asp Asp Thr Glu

            20                  25                  30

Arg Leu Pro Ser Lys Cys Glu Val Cys Lys Leu Leu Ser Thr Glu Leu

        35                  40                  45

Gln Ala Glu Leu Ser Arg Thr Gly Arg Ser Arg Glu Val Leu Glu Leu

    50                  55                  60

Gly Gln Val Leu Asp Thr Gly Lys Arg Lys Arg His Val Pro Tyr Ser

65                  70                  75                  80

Val Ser Glu Thr Arg Leu Glu Glu Ala Leu Glu Asn Leu Cys Glu Arg

                85                  90                  95

Ile Leu Asp Tyr Ser Val His Ala Glu Arg Lys Gly Ser Leu Arg Tyr

            100                 105                 110

Ala Lys Gly Gln Ser Gln Thr Met Ala Thr Leu Lys Gly Leu Val Gln

        115                 120                 125

Lys Gly Val Lys Val Asp Leu Gly Ile Pro Leu Glu Leu Trp Asp Glu

    130                 135                 140

Pro Ser Val Glu Val Thr Tyr Leu Lys Lys Gln Cys Glu Thr Met Leu

145                 150                 155                 160

Glu Glu Phe Glu Asp Ile Val Gly Asp Trp Tyr Phe His His Gln Glu

                165                 170                 175

Gln Pro Leu Gln Asn Phe Leu Cys Glu Gly His Val Leu Pro Ala Ala

            180                 185                 190

Glu Thr Ala Cys Leu Gln Glu Thr Trp Thr Gly Lys Glu Ile Thr Asp

        195                 200                 205

Gly Glu Glu Lys Thr Glu Gly Glu Glu Glu Gln Glu Glu Glu Glu Glu
    210                 215                 220
Glu Glu Glu Glu Glu Gly Gly Asp Lys Met Thr Lys Thr Gly Ser His
225                 230                 235                 240
Pro Lys Leu Asp Arg Glu Asp Leu
                245
<210> 5
<211> 173
<212> PRT
<213> Human
<400> 5
Met Phe Cys Pro Leu Lys Leu Ile Leu Leu Pro Val Leu Leu Asp Tyr
                5                   10                  15
Ser Leu Gly Leu Asn Asp Leu Asn Val Ser Pro Pro Glu Leu Thr Val
            20                  25                  30
His Val Gly Asp Ser Ala Leu Met Gly Cys Val Phe Gln Ser Thr Glu
        35                  40                  45
Asp Lys Cys Ile Phe Lys Ile Asp Trp Thr Leu Ser Pro Gly Glu His
    50                  55                  60
Ala Lys Asp Glu Tyr Val Leu Tyr Tyr Tyr Ser Asn Leu Ser Val Pro
65                  70                  75                  80
Ile Gly Arg Phe Gln Asn Arg Val His Leu Met Gly Asp Ile Leu Cys
                85                  90                  95
Asn Asp Gly Ser Leu Leu Leu Gln Asp Val Gln Glu Ala Asp Gln Gly
            100                 105                 110
Thr Tyr Ile Cys Glu Ile Arg Leu Lys Gly Glu Ser Gln Val Phe Lys
        115                 120                 125
Lys Ala Val Val Leu His Val Leu Pro Glu Glu Pro Lys Glu Leu Met

    130                 135                 140
Val His Val Gly Gly Leu Ile Gln Met Gly Cys Val Phe Gln Ser Thr
145                 150                 155                 160
Glu Val Lys His Val Thr Lys Val Glu Trp Ile Phe Ser
                165                 170
<210> 6
<211> 261
<212> PRT
<213> Human
<400> 6
Met Glu Leu Leu Gln Val Thr Ile Leu Phe Leu Leu Pro Ser Ile Cys
                5                   10                  15
Ser Ser Asn Ser Thr Gly Val Leu Glu Ala Ala Asn Asn Ser Leu Val
            20                  25                  30
Val Thr Thr Thr Lys Pro Ser Ile Thr Thr Pro Asn Thr Glu Ser Leu
        35                  40                  45
Gln Lys Asn Val Val Thr Pro Thr Thr Gly Thr Thr Pro Lys Gly Thr
    50                  55                  60
Ile Thr Asn Glu Leu Leu Lys Met Ser Leu Met Ser Thr Ala Thr Phe
65                  70                  75                  80
Leu Thr Ser Lys Asp Glu Gly Leu Lys Ala Thr Thr Thr Asp Val Arg
                85                  90                  95
Lys Asn Asp Ser Ile Ile Ser Asn Val Thr Val Thr Ser Val Thr Leu
            100                 105                 110
Pro Asn Ala Val Ser Thr Leu Gln Ser Ser Lys Pro Lys Thr Glu Thr
        115                 120                 125
Gln Ser Ser Ile Lys Thr Thr Glu Ile Pro Gly Ser Val Leu Gln Pro
    130                 135                 140

Asp Ala Ser Pro Ser Lys Thr Gly Thr Leu Thr Ser Ile Pro Val Thr
145                 150                 155                 160
Ile Pro Glu Asn Thr Ser Gln Ser Gln Val Ile Gly Thr Glu Gly Gly
                165                 170                 175
Lys Asn Ala Ser Thr Ser Ala Thr Ser Arg Ser Tyr Ser Ser Ile Ile
            180                 185                 190
Leu Pro Val Val Ile Ala Leu Ile Val Ile Thr Leu Ser Val Phe Val
        195                 200                 205
Leu Val Gly Leu Tyr Arg Met Cys Trp Lys Ala Asp Pro Gly Thr Pro
    210                 215                 220
Glu Asn Gly Asn Asp Gln Pro Gln Ser Asp Lys Glu Ser Val Lys Leu
225                 230                 235                 240
Leu Thr Val Lys Thr Ile Ser His Glu Ser Gly Glu His Ser Ala Gln
                245                 250                 255
Gly Lys Thr Lys Asn
            260
<210> 7
<211> 243
<212> PRT
<213> Human
<400> 7

Met Arg Pro Gln Gly Pro Ala Ala Ser Pro Gln Arg Leu Arg Gly Leu
                5                   10                  15
Leu Leu Leu Leu Leu Leu Gln Leu Pro Ala Pro Ser Ser Ala Ser Glu
            20                  25                  30
Ile Pro Lys Gly Lys Gln Lys Ala Gln Leu Arg Gln Arg Glu Val Val
        35                  40                  45
Asp Leu Tyr Asn Gly Met Cys Leu Gln Gly Pro Ala Gly Val Pro Gly

    50                  55                  60
Arg Asp Gly Ser Pro Gly Ala Asn Gly Ile Pro Gly Thr Pro Gly Ile
65                  70                  75                  80
Pro Gly Arg Asp Gly Phe Lys Gly Glu Lys Gly Glu Cys Leu Arg Glu
                85                  90                  95
Ser Phe Glu Glu Ser Trp Thr Pro Asn Tyr Lys Gln Cys Ser Trp Ser
            100                 105                 110
Ser Leu Asn Tyr Gly Ile Asp Leu Gly Lys Ile Ala Glu Cys Thr Phe
        115                 120                 125
Thr Lys Met Arg Ser Asn Ser Ala Leu Arg Val Leu Phe Ser Gly Ser
    130                 135                 140
Leu Arg Leu Lys Cys Arg Asn Ala Cys Cys Gln Arg Trp Tyr Phe Thr
145                 150                 155                 160
Phe Asn Gly Ala Glu Cys Ser Gly Pro Leu Pro Ile Glu Ala Ile Ile
                165                 170                 175
Tyr Leu Asp Gln Gly Ser Pro Glu Met Asn Ser Thr Ile Asn Ile His
            180                 185                 190
Arg Thr Ser Ser Val Glu Gly Leu Cys Glu Gly Ile Gly Ala Gly Leu
        195                 200                 205
Val Asp Val Ala Ile Trp Val Gly Thr Cys Ser Asp Tyr Pro Lys Gly
    210                 215                 220
Asp Ala Ser Thr Gly Trp Asn Ser Val Ser Arg Ile Ile Ile Glu Glu
225                 230                 235                 240
Leu Pro Lys
<210> 8
<211> 149
<212> PRT
<213> Human
<400> 8

Met Lys Leu Gln Cys Val Ser Leu Trp Leu Leu Gly Thr Ile Leu Ile
                5                   10                  15
Leu Cys Ser Val Asp Asn His Gly Leu Arg Arg Cys Leu Ile Ser Thr
            20                  25                  30
Asp Met His His Ile Glu Glu Ser Phe Gln Glu Ile Lys Arg Ala Ile
        35                  40                  45
Gln Ala Lys Asp Thr Phe Pro Asn Val Thr Ile Leu Ser Thr Leu Glu
    50                  55                  60
Thr Leu Gln Ile Ile Lys Pro Leu Asp Val Cys Cys Val Thr Lys Asn
65                  70                  75                  80
Leu Leu Ala Phe Tyr Val Asp Arg Val Phe Lys Asp His Gln Glu Pro
                85                  90                  95
Asn Pro Lys Ile Leu Arg Lys Ile Ile Ser Ile Cys Gln Leu Phe Pro
            100                 105                 110
Leu His Ala Glu Asn Ser Ala Ala Met Cys Glu Ser Leu Gly Gln Asn
        115                 120                 125
Ser Ser Ile Cys Ser Leu Ser Ala Gln Gly Glu Ala Arg Lys Cys Trp
    130                 135                 140
Pro Pro Ser Ala Ser
145
<210> 9
<211> 136
<212> PRT
<213> Human
<400> 9
Met Ala Ser Leu Gly Leu Leu Leu Leu Leu Leu Leu Thr Ala Leu Pro
                5                   10                  15
Pro Leu Trp Ser Ser Ser Leu Pro Gly Leu Asp Thr Ala Glu Ser Lys
            20                  25                  30

Ala Thr Ile Ala Asp Leu Ile Leu Ser Ala Leu Glu Arg Ala Thr Val
        35                  40                  45
Phe Leu Glu Gln Arg Leu Pro Glu Ile Asn Leu Asp Gly Met Val Gly
    50                  55                  60
Val Arg Val Leu Glu Glu Gln Leu Lys Ser Val Arg Glu Lys Trp Ala
65                  70                  75                  80
Gln Glu Pro Leu Leu Gln Pro Leu Ser Leu Arg Val Gly Met Leu Gly
                85                  90                  95
Glu Lys Leu Glu Ala Ala Ile Gln Arg Ser Leu His Tyr Leu Lys Leu
            100                 105                 110
Ser Asp Pro Lys Tyr Leu Arg Gly Arg Thr Ala Ala Ser Pro Ala Ala
        115                 120                 125
Ser Gln Thr Ser Ala Gly Ala Ser
    130                 135
<210> 10
<211> 123
<212> PRT
<213> Human
<400> 10
Met Lys Leu Leu Leu Leu Ala Leu Pro Met Leu Val Leu Leu Pro Gln
                5                   10                  15
Val Ile Pro Ala Tyr Ser Gly Glu Lys Lys Cys Trp Asn Arg Ser Gly
            20                  25                  30
His Cys Arg Lys Gln Cys Lys Asp Gly Glu Ala Val Lys Asp Thr Cys
        35                  40                  45
Lys Asn Leu Arg Ala Cys Cys Ile Pro Ser Asn Glu Asp His Arg Arg
    50                  55                  60
Val Pro Ala Thr Ser Pro Thr Pro Leu Ser Asp Ser Thr Pro Gly Ile
65                  70                  75                  80

Ile Asp Asp Ile Leu Thr Val Arg Phe Thr Thr Asp Tyr Phe Glu Val
                85                  90                  95
Ser Ser Lys Lys Asp Met Val Glu Glu Ser Glu Ala Gly Arg Gly Thr
            100                 105                 110
Glu Thr Ser Leu Pro Asn Val His His Ser Ser
        115                 120
<210> 11
<211> 163
<212> PRT
<213> Human
<400> 11
Met Gly Gly Leu Leu Leu Ala Ala Phe Leu Ala Leu Val Ser Val Pro
                5                   10                  15
Arg Ala Gln Ala Val Trp Leu Gly Arg Leu Asp Pro Glu Gln Leu Leu
            20                  25                  30
Gly Pro Trp Tyr Val Leu Ala Val Ala Ser Arg Glu Lys Gly Phe Ala
        35                  40                  45
Met Glu Lys Asp Met Lys Asn Val Val Gly Val Val Val Thr Leu Thr
    50                  55                  60
Pro Glu Asn Asn Leu Arg Thr Leu Ser Ser Gln His Gly Leu Gly Gly
65                  70                  75                  80
Cys Asp Gln Ser Val Met Asp Leu Ile Lys Arg Asn Ser Gly Trp Val
                85                  90                  95
Phe Glu Asn Pro Ser Ile Gly Val Leu Glu Leu Trp Val Leu Ala Thr
            100                 105                 110
Asn Phe Arg Asp Tyr Ala Ile Ile Phe Thr Gln Leu Glu Phe Gly Asp
        115                 120                 125
Glu Pro Phe Asn Thr Val Glu Leu Tyr Ser Leu Thr Glu Thr Ala Ser

    130                 135                 140
Gln Glu Ala Met Gly Leu Phe Thr Lys Trp Ser Arg Ser Leu Gly Phe
145                 150                 155                 160
Leu Ser Gln
<210> 12
<211> 301
<212> PRT
<213> Human
<400> 12
Met Ala Arg His Gly Leu Pro Leu Leu Pro Leu Leu Ser Leu Leu Val
                5                   10                  15
Gly Ala Trp Leu Lys Leu Gly Asn Gly Gln Ala Thr Ser Met Val Gln
            20                  25                  30
Leu Gln Gly Gly Arg Phe Leu Met Gly Thr Asn Ser Pro Asp Ser Arg
        35                  40                  45
Asp Gly Glu Gly Pro Val Arg Glu Ala Thr Val Lys Pro Phe Ala Ile
    50                  55                  60
Asp Ile Phe Pro Val Thr Asn Lys Asp Phe Arg Asp Phe Val Arg Glu
65                  70                  75                  80
Lys Lys Tyr Arg Thr Glu Ala Glu Met Phe Gly Trp Ser Phe Val Phe
                85                  90                  95
Glu Asp Phe Val Ser Asp Glu Leu Arg Asn Lys Ala Thr Gln Pro Met
            100                 105                 110
Lys Ser Val Leu Trp Trp Leu Pro Val Glu Lys Ala Phe Trp Arg Gln
        115                 120                 125
Pro Ala Gly Pro Gly Ser Gly Ile Arg Glu Arg Leu Glu His Pro Val
    130                 135                 140
Leu His Val Ser Trp Asn Asp Ala Arg Ala Tyr Cys Ala Trp Arg Gly
145                 150                 155                 160

Lys Arg Leu Pro Thr Glu Glu Glu Trp Glu Phe Ala Ala Arg Gly Gly
                165                 170                 175
Leu Lys Gly Gln Val Tyr Pro Trp Gly Asn Trp Phe Gln Pro Asn Arg
            180                 185                 190
Thr Asn Leu Trp Gln Gly Lys Phe Pro Lys Gly Asp Lys Ala Glu Asp
        195                 200                 205
Gly Phe His Gly Val Ser Pro Val Asn Ala Phe Pro Ala Gln Asn Asn
    210                 215                 220
Tyr Gly Leu Tyr Asp Leu Leu Gly Asn Val Trp Glu Trp Thr Ala Ser
225                 230                 235                 240
Pro Tyr Gln Ala Ala Glu Gln Asp Met Arg Val Leu Arg Gly Ala Ser
                245                 250                 255
Trp Ile Asp Thr Ala Asp Gly Ser Ala Asn His Arg Ala Arg Val Thr
            260                 265                 270
Thr Arg Met Gly Asn Thr Pro Asp Ser Ala Ser Asp Asn Leu Gly Phe
        275                 280                 285
Arg Cys Ala Ala Asp Ala Gly Arg Pro Pro Gly Glu Leu
    290                 295                 300
<210> 13
<211> 69
<212> PRT
<213> Human
<400> 13
Met Cys Trp Leu Arg Ala Trp Gly Gln Ile Leu Leu Pro Val Phe Leu
                5                   10                  15
Ser Leu Phe Leu Ile Gln Leu Leu Ile Ser Phe Ser Glu Asn Gly Phe
            20                  25                  30
Ile His Ser Pro Arg Asn Asn Gln Lys Pro Arg Asp Gly Asn Glu Glu
        35                  40                  45

Glu Cys Ala Val Lys Lys Ser Cys Gln Leu Cys Thr Glu Asp Lys Lys
    50                  55                  60
Tyr Met Met Asn Arg
65
<210> 14
<211> 69
<212> PRT
<213> Human
<400> 14
Met Gly Phe Pro Ala Ala Ala Leu Leu Cys Ala Leu Cys Cys Gly Leu
                5                   10                  15
Leu Ala Pro Ala Ala Arg Ala Gly Tyr Ser Glu Glu Arg Cys Ser Trp
            20                  25                  30
Arg Gly Arg Pro Arg Arg Thr Arg Thr Ser Ala Ala Ala Trp Pro Pro
        35                  40                  45
Ser Ala Leu Ser Cys Ala Arg Thr Gly Ala Pro Ser Cys Pro Arg Arg
    50                  55                  60
Pro Thr Val Ser Ala
65
<210> 15
<211> 197
<212> PRT
<213> Human
<400> 15
Met Arg Gly Gly Lys Cys Asn Met Leu Ser Ser Leu Gly Cys Leu Leu
                5                   10                  15
Leu Cys Gly Ser Ile Thr Leu Ala Leu Gly Asn Ala Gln Lys Leu Pro
            20                  25                  30
Lys Gly Lys Arg Pro Asn Leu Lys Val His Ile Asn Thr Thr Ser Asp

        35                  40                  45
Ser Ile Leu Leu Lys Phe Leu Arg Pro Ser Pro Asn Val Lys Leu Glu
    50                  55                  60
Gly Leu Leu Leu Gly Tyr Gly Ser Asn Val Ser Pro Asn Gln Tyr Phe
65                  70                  75                  80
Pro Leu Pro Ala Glu Gly Lys Phe Thr Glu Ala Ile Val Asp Ala Glu
                85                  90                  95
Pro Lys Tyr Leu Ile Val Val Arg Pro Ala Pro Pro Pro Ser Gln Lys
            100                 105                 110
Lys Ser Cys Ser Gly Lys Thr Arg Ser Arg Lys Pro Leu Gln Leu Val
        115                 120                 125
Val Gly Thr Leu Thr Pro Ser Ser Val Phe Leu Ser Trp Gly Phe Leu
    130                 135                 140
Ile Asn Pro His His Asp Trp Thr Leu Pro Ser His Cys Pro Asn Asp
145                 150                 155                 160
Arg Phe Tyr Thr Ile Arg Tyr Arg Glu Lys Asp Lys Glu Lys Lys Trp
                165                 170                 175
Ile Phe Gln Ile Cys Pro Ala Thr Glu Thr Ile Val Glu Asn Leu Lys
            180                 185                 190
Pro Asn Thr Ser Leu
        195
<210> 16
<211> 378
<212> DNA
<213> Human
<400> 16
ATGGCCAAGT ACCTGGCCCA GATCATTGTG ATGGGCGTGC AGGTGGTGGG CAGGGCCTTT 60
GCACGGGCCT TGCGGCAGGA GTTTGCAGCC AGCCGGGCCG CAGCTGATGC CCGAGGACGC 120

GCTGGACACC GGTCTGCAGC CGCTTCCAAC CTCTCCGGCC TCAGCCTCCA GGAGGCACAG 180
CAGATTCTCA ACGTGTCCAA GCTGAGCCCT GAGCAGGTCC AGAAGAACTA TGAACACTTA 240
TTTAAGGTGA ATGATAAATC CGTGGGTGGC TCCTTCTACC TGCAGTCAAA GGTGGTCCGC 300
GCAAAGGAGC GCCTGGATGA GGAACTCAAA ATCCAGGCCC AGGAGGACAG AGAAAAAGGG 360
CAGATGCCCC ATACGTGA 378
<210> 17
<211> 366
<212> DNA
<213> Human
<400> 17
ATGCACAGAT CAGAGCCATT TCTGAAAATG TCGCTGCTGA TTCTGCTTTT CCTGGGATTG 60
GCAGAAGCCT GTACTCCTCG TGAAGTCAAC TTGCTGAAAG GGATCATAGG TCTCATGAGC 120
AGACTGTCAC CGGATGAGAT CCTAGGCTTG CTGAGCCTCC AAGTACTGCA TGAAGAAACA 180
AGTGGCTGCA AGGAGGAAGT TAAACCCTTC TCAGGCACCA CCCCATCCAG GAAACCACTC 240
CCCAAGAGGA AGAACACGTG GAACTTCCTG AAATGCGCCT ACATGGTGAT GACCTACCTC 300
TTCGTATCCT ACAACAAAGG GGACTGGTTC ACTTTTTCCT CCCAAGTGTT ACTGCCACTA 360
CTGTAA 366
<210> 18
<211> 672
<212> DNA
<213> Human
<400> 18
ATGAAGTTCG TCCCCTGCCT CCTGCTGGTG ACCTTGTCCT GCCTGGGGAC TTTGGGTCAG 60
GCCCCGAGGC AAAAGCAAGG AAGCACTGGG GAGGAATTCC ATTTCCAGAC TGGAGGGAGA 120
GATTCCTGCA CTATGCGTCC CAGCAGCTTG GGGCAAGGTG CTGGAGAAGT CTGGCTTCGC 180
GTCGACTGCC GCAACACAGA CCAGACCTAC TGGTGTGAGT ACAGGGGGCA GCCCAGCATG 240
TGCCAGGCTT TCGCTGCTGA CCCCAAATCT TACTGGAATC AAGCCCTGCA GGAGCTGAGG 300
CGCCTTCACC ATGCGTGCCA GGGGGCCCCG GTGCTTAGGC CATCCGTGTG CAGGGAGGCT 360
GGACCCCAGG CCCATATGCA GCAGGTGACT TCCAGCCTCA AGGGCAGCCC AGAGCCCAAC 420

CAGCAGCCTG AGGCTGGGAC GCCATCTCTG AGCCCCAAGG CCACAGTGAA ACTCACAGGA 480
GCAACACAGC TGGGAAAGGA CTCGATGGAA GAGCTGGGAA AAGCCAAACC CACCACCGGA 540
CCCACAGCCA AACCTACCCA GCCTGGACCC AGGCCCGGAG GGAATGAGGA AGCAAAGAAG 600
AAGGCCTGGG AACATTGTTG GAAACCCTTC CAGGCCCTGT GCGCCTTTCT CATCAGCTTC 660
TTCCGAGGGT GA 672
<210> 19
<211> 747
<212> DNA
<213> Human
<400> 19
ATGGGACCTG TGCGGTTGGG AATATTGCTT TTCCTTTTTT TGGCCGTGCA CGAGGCTTGG 60
GCTGGGATGT TGAAGGAGGA GGACGATGAC ACAGAACGCT TGCCCAGCAA ATGCGAAGTG 120
TGTAAGCTGC TGAGCACAGA GCTACAGGCG GAACTGAGTC GCACCGGTCG ATCTCGAGAG 180
GTGCTGGAGC TGGGGCAGGT GCTGGATACA GGCAAGAGGA AGAGACACGT GCCTTACAGC 240
GTTTCAGAGA CAAGGCTGGA AGAGGCCTTA GAGAATTTAT GTGAGCGGAT CCTGGACTAT 300
AGTGTTCACG CTGAGCGCAA GGGCTCACTG AGATATGCCA AGGGTCAGAG TCAGACCATG 360
GCAACACTGA AAGGCCTAGT GCAGAAGGGG GTGAAGGTGG ATCTGGGGAT CCCTCTGGAG 420
CTTTGGGATG AGCCCAGCGT GGAGGTCACA TACCTCAAGA AGCAGTGTGA GACCATGTTG 480
GAGGAGTTTG AAGACATTGT GGGAGACTGG TACTTCCACC ATCAGGAGCA GCCCCTACAA 540
AATTTTCTCT GTGAAGGTCA TGTGCTCCCA GCTGCTGAAA CTGCATGTCT ACAGGAAACT 600
TGGACTGGAA AGGAGATCAC AGATGGGGAA GAGAAAACAG AAGGGGAGGA AGAGCAGGAG 660
GAGGAGGAGG AAGAGGAGGA AGAGGAAGGG GGAGACAAGA TGACCAAGAC AGGAAGCCAC 720
CCCAAACTTG ACCGAGAAGA TCTTTGA 747
<210> 20
<211> 522
<212> DNA
<213> Human
<400> 20
ATGTTTTGCC CACTGAAACT CATCCTGCTG CCAGTGTTAC TGGATTATTC CTTGGGCCTG 60

AATGACTTGA ATGTTTCCCC GCCTGAGCTA ACAGTCCATG TGGGTGATTC AGCTCTGATG 120
GGATGTGTTT TCCAGAGCAC AGAAGACAAA TGTATATTCA AGATAGACTG GACTCTGTCA 180
CCAGGAGAGC ACGCCAAGGA CGAATATGTG CTATACTATT ACTCCAATCT CAGTGTGCCT 240
ATTGGGCGCT TCCAGAACCG CGTACACTTG ATGGGGGACA TCTTATGCAA TGATGGCTCT 300
CTCCTGCTCC AAGATGTGCA AGAGGCTGAC CAGGGAACCT ATATCTGTGA AATCCGCCTC 360
AAAGGGGAGA GCCAGGTGTT CAAGAAGGCG GTGGTACTGC ATGTGCTTCC AGAGGAGCCC 420
AAAGAGCTCA TGGTCCATGT GGGTGGATTG ATTCAGATGG GATGTGTTTT CCAGAGCACA 480
GAAGTGAAAC ACGTGACCAA GGTAGAATGG ATATTTTCAT GA 522
<210> 21
<211> 786
<212> DNA
<213> Human
<400> 21
ATGGAACTGC TTCAAGTGAC CATTCTTTTT CTTCTGCCCA GTATTTGCAG CAGTAACAGC 60
ACAGGTGTTT TAGAGGCAGC TAATAATTCA CTTGTTGTTA CTACAACAAA ACCATCTATA 120
ACAACACCAA ACACAGAATC ATTACAGAAA AATGTTGTCA CACCAACAAC TGGAACAACT 180
CCTAAAGGAA CAATCACCAA TGAATTACTT AAAATGTCTC TGATGTCAAC AGCTACTTTT 240
TTAACAAGTA AAGATGAAGG ATTGAAAGCC ACAACCACTG ATGTCAGGAA GAATGACTCC 300
ATCATTTCAA ACGTAACAGT AACAAGTGTT ACACTTCCAA ATGCTGTTTC AACATTACAA 360
AGTTCCAAAC CCAAGACTGA AACTCAGAGT TCAATTAAAA CAACAGAAAT ACCAGGTAGT 420
GTTCTACAAC CAGATGCATC ACCTTCTAAA ACTGGTACAT TAACCTCAAT ACCAGTTACA 480
ATTCCAGAAA ACACCTCACA GTCTCAAGTA ATAGGCACTG AGGGTGGAAA AAATGCAAGC 540
ACTTCAGCAA CCAGCCGGTC TTATTCCAGT ATTATTTTGC CGGTGGTTAT TGCTTTGATT 600
GTAATAACAC TTTCAGTATT TGTTCTGGTG GGTTTGTACC GAATGTGCTG GAAGGCAGAT 660
CCGGGCACAC CAGAAAATGG AAATGATCAA CCTCAGTCTG ATAAAGAGAG CGTGAAGCTT 720
CTTACCGTTA AGACAATTTC TCATGAGTCT GGTGAGCACT CTGCACAAGG AAAAACCAAG 780
AACTGA 786
<210> 22
<211> 732

<212> DNA

<213> Human

<400> 22

ATGCGACCCC AGGGCCCCGC CGCCTCCCCG CAGCGGCTCC GCGGCCTCCT GCTGCTCCTG 60

CTGCTGCAGC TGCCCGCGCC GTCGAGCGCC TCTGAGATCC CCAAGGGGAA GCAAAAGGCG 120

CAGCTCCGGC AGAGGGAGGT GGTGGACCTG TATAATGGAA TGTGCTTACA AGGGCCAGCA 180

GGAGTGCCTG GTCGAGACGG GAGCCCTGGG GCCAATGGCA TTCCGGGTAC ACCTGGGATC 240

CCAGGTCGGG ATGGATTCAA AGGAGAAAAG GGGGAATGTC TGAGGGAAAG CTTTGAGGAG 300

TCCTGGACAC CCAACTACAA GCAGTGTTCA TGGAGTTCAT TGAATTATGG CATAGATCTT 360

GGGAAAATTG CGGAGTGTAC ATTTACAAAG ATGCGTTCAA ATAGTGCTCT AAGAGTTTTG 420

TTCAGTGGCT CACTTCGGCT AAAATGCAGA AATGCATGCT GTCAGCGTTG GTATTTCACA 480

TTCAATGGAG CTGAATGTTC AGGACCTCTT CCCATTGAAG CTATAATTTA TTTGGACCAA 540

GGAAGCCCTG AAATGAATTC AACAATTAAT ATTCATCGCA CTTCTTCTGT GGAAGGACTT 600

TGTGAAGGAA TTGGTGCTGG ATTAGTGGAT GTTGCTATCT GGGTTGGCAC TTGTTCAGAT 660

TACCCAAAAG GAGATGCTTC TACTGGATGG AATTCAGTTT CTCGCATCAT TATTGAAGAA 720

CTACCAAAAT AA 732

<210> 23

<211> 450

<212> DNA

<213> Human

<400> 23

ATGAAGTTAC AGTGTGTTTC CCTTTGGCTC CTGGGTACAA TACTGATATT GTGCTCAGTA 60

GACAACCACG GTCTCAGGAG ATGTCTGATT TCCACAGACA TGCACCATAT AGAAGAGAGT 120

TTCCAAGAAA TCAAAAGAGC CATCCAAGCT AAGGACACCT TCCCAAATGT CACTATCCTG 180

TCCACATTGG AGACTCTGCA GATCATTAAG CCCTTAGATG TGTGCTGCGT GACCAAGAAC 240

CTCCTGGCGT TCTACGTGGA CAGGGTGTTC AAGGATCATC AGGAGCCAAA CCCCAAAATC 300

TTGAGAAAAA TCATCAGCAT TTGCCAACTC TTTCCTCTAC ATGCAGAAAA CTCTGCGGCA 360

ATGTGTGAGT CACTGGGTCA GAATTCCAGC ATCTGCTCCC TGTCTGCCCA AGGAGAGGCC 420

AGGAAGTGCT GGCCCCCATC GGCCTCCTGA 450

<210> 24

<211> 411

<212> DNA

<213> Human

<400> 24

ATGGCCAGCC TGGGGCTGCT GCTCCTGCTC TTACTGACAG CACTGCCACC GCTGTGGTCC 60

TCCTCACTGC CTGGGCTGGA CACTGCTGAA AGTAAAGCCA CCATTGCAGA CCTGATCCTG 120

TCTGCGCTGG AGAGAGCCAC CGTCTTCCTA GAACAGAGGC TGCCTGAAAT CAACCTGGAT 180

GGCATGGTGG GGGTCCGAGT GCTGGAAGAG CAGCTAAAAA GTGTCCGGGA GAAGTGGGCC 240

CAGGAGCCCC TGCTGCAGCC GCTGAGCCTG CGCGTGGGGA TGCTGGGGGA GAAGCTGGAG 300

GCTGCCATCC AGAGATCCCT CCACTACCTC AAGCTGAGTG ATCCCAAGTA CCTAAGAGGA 360

CGGACAGCAG CGAGCCCTGC GGCCTCTCAG ACCTCTGCAG GAGCCTCATG A 411

<210> 25

<211> 372

<212> DNA

<213> Human

<400> 25

ATGAAACTCC TGCTGCTGGC TCTTCCTATG CTTGTGCTCC TACCCCAAGT GATCCCAGCC 60

TATAGTGGTG AAAAAAAATG CTGGAACAGA TCAGGGCACT GCAGGAAACA ATGCAAAGAT 120

GGAGAAGCAG TGAAAGATAC ATGCAAAAAT CTTCGAGCTT GCTGCATTCC ATCCAATGAA 180

GACCACAGGC GAGTTCCTGC GACATCTCCC ACACCCTTGA GTGACTCAAC ACCAGGAATT 240

ATTGATGATA TTTTAACAGT AAGGTTCACG ACAGACTACT TTGAAGTAAG CAGCAAGAAA 300

GATATGGTTG AAGAGTCTGA GGCGGGAAGG GGAACTGAGA CCTCTCTTCC AAATGTTCAC 360

CATAGCTCAT GA 372

<210> 26

<211> 492

<212> DNA

<213> Human

<400> 26

ATGGGCGGCC TGCTGCTGGC TGCTTTTCTG GCTTTGGTCT CGGTGCCCAG GGCCCAGGCC 60
GTGTGGTTGG GAAGACTGGA CCCTGAGCAG CTTCTTGGGC CCTGGTACGT GCTTGCGGTG 120
GCCTCCCGGG AAAAGGGCTT TGCCATGGAG AAGGACATGA AGAACGTCGT GGGGGTGGTG 180
GTGACCCTCA CTCCAGAAAA CAACCTGCGG ACGCTGTCCT CTCAGCACGG GCTGGGAGGG 240
TGTGACCAGA GTGTCATGGA CCTGATAAAG CGAAACTCCG GATGGGTGTT TGAGAATCCC 300
TCAATAGGCG TGCTGGAGCT CTGGGTGCTG GCCACCAACT TCAGAGACTA TGCCATCATC 360
TTCACTCAGC TGGAGTTCGG GGACGAGCCC TTCAACACCG TGGAGCTGTA CAGTCTGACG 420
GAGACAGCCA GCCAGGAGGC CATGGGGCTC TTCACCAAGT GGAGCAGGAG CCTGGGCTTC 480
CTGTCACAGT AG 492
<210> 27
<211> 906
<212> DNA
<213> Human
<400> 27
ATGGCCCGGC ATGGGTTACC GCTGCTGCCC CTGCTGTCGC TCCTGGTCGG CGCGTGGCTC 60
AAGCTAGGAA ATGGACAGGC TACTAGCATG GTCCAACTGC AGGGTGGGAG ATTCCTGATG 120
GGAACAAATT CTCCAGACAG CAGAGATGGT GAAGGGCCTG TGCGGGAGGC GACAGTGAAA 180
CCCTTTGCCA TCGACATATT TCCTGTCACC AACAAAGATT TCAGGGATTT TGTCAGGGAG 240
AAAAAGTATC GGACAGAAGC TGAGATGTTT GGATGGAGCT TTGTCTTTGA GGACTTTGTC 300
TCTGATGAGC TGAGAAACAA AGCCACCCAG CCAATGAAGT CTGTACTCTG GTGGCTTCCA 360
GTGGAAAAGG CATTTTGGAG GCAGCCTGCA GGTCCTGGCT CTGGCATCCG AGAGAGACTG 420
GAGCACCCAG TGTTACACGT GAGCTGGAAT GACGCCCGTG CCTACTGTGC TTGGCGGGGA 480
AAACGACTGC CCACGGAGGA AGAGTGGGAG TTTGCCGCCC GAGGGGGCTT GAAGGGTCAA 540
GTTTACCCAT GGGGGAACTG GTTCCAGCCA AACCGCACCA ACCTGTGGCA GGGAAAGTTC 600
CCCAAGGGAG ACAAAGCTGA GGATGGCTTC CATGGAGTCT CCCCAGTGAA TGCTTTCCCC 660
GCCCAGAACA ACTACGGGCT CTATGACCTC CTGGGGAACG TGTGGGAGTG GACAGCATCA 720
CCGTACCAGG CTGCTGAGCA GGACATGCGC GTCCTCCGGG GGGCATCCTG GATCGACACA 780
GCTGATGGCT CTGCCAATCA CCGGGCCCGG GTCACCACCA GGATGGGCAA CACTCCAGAT 840
TCAGCCTCAG ACAACCTCGG TTTCCGCTGT GCTGCAGACG CAGGCCGGCC GCCAGGGGAG 900

CTGTAA 906
<210> 28
<211> 210
<212> DNA
<213> Human
<400> 28
ATGTGCTGGC TGCGGGCATG GGGCCAGATC CTCCTGCCAG TTTTCCTCTC CCTCTTTCTC 60
ATCCAATTGC TTATCAGCTT CTCAGAGAAT GGTTTTATCC ACAGCCCCAG GAACAATCAG 120
AAACCAAGAG ATGGGAATGA AGAGGAATGT GCTGTAAAGA AGAGTTGTCA ATTGTGCACA 180
GAAGATAAGA AATATATGAT GAATAGATAA 210
<210> 29
<211> 210
<212> DNA
<213> Human
<400> 29
ATGGGGTTCC CGGCCGCGGC GCTGCTCTGC GCGCTGTGCT GCGGCCTCCT GGCCCCGGCT 60
GCCCGCGCCG GCTACTCCGA GGAGCGCTGC AGCTGGAGGG GCAGGCCACG CCGCACCAGG 120
ACATCAGCCG CCGCGTGGCC GCCTTCCGCT TTGAGCTGCG CGAGGACGGG CGCCCCGAGC 180
TGCCCCCGCA GGCCCACGGT CTCGGCGTAG 210
<210> 30
<211> 594
<212> DNA
<213> Human
<400> 30
ATGCGAGGTG GCAAATGCAA CATGCTCTCC AGTTTGGGGT GTCTACTTCT CTGTGGAAGT 60
ATTACACTAG CCCTGGGAAA TGCACAGAAA TTGCCAAAAG GTAAAAGGCC AAACCTCAAA 120
GTCCACATCA ATACCACAAG TGACTCCATC CTCTTGAAGT TCTTGCGTCC AAGTCCAAAT 180
GTAAAGCTTG AAGGTCTTCT CCTGGGATAT GGCAGCAATG TATCACCAAA CCAGTACTTC 240
CCTCTTCCCG CTGAAGGGAA ATTCACAGAA GCTATAGTTG ATGCAGAGCC GAAATATCTG 300

ATAGTTGTGC GACCTGCTCC ACCTCCAAGT CAAAAGAAGT CATGTTCAGG TAAAACTCGT 360
TCTCGCAAAC CTCTGCAGCT GGTGGTTGGC ACTCTGACAC CGAGCTCGGT CTTCCTGTCC 420
TGGGGTTTCC TCATCAACCC ACACCATGAC TGGACATTGC CAAGTCACTG TCCCAATGAC 480
AGATTTTATA CAATTCGCTA TCGAGAAAAG GATAAAGAAA AGAAGTGGAT TTTTCAAATC 540
TGTCCAGCCA CTGAAACAAT TGTGGAAAAC CTAAAGCCCA ACACAAGTTT ATGA 594
<210> 31
<211> 25
<212> DNA
<213> Artificial Sequence
<220>
<223>
<400> 31
ATGGCCAAGT ACCTGGCCCA GATCA 25
<210> 32
<211> 25
<212> DNA
<213> Artificial Sequence
<220>
<223>
<400> 32
TCACGTATGG GGCATCTGCC CTTTT 25
<210> 33
<211> 25
<212> DNA
<213> Artificial Sequence
<220>
<223>
<400> 33
ATGCACAGAT CAGAGCCATT TCTGA 25

<210> 34

<211> 25

<212> DNA

<213> Artificial Sequence

<220>

<223>

<400> 34

TTACAGTAGT GGCAGTAACA CTTGG 25

<210> 35

<211> 25

<212> DNA

<213> Artificial Sequence

<220>

<223>

<400> 35

ATGAAGTTCG TCCCCTGCCT CCTGC 25

<210> 36

<211> 25

<212> DNA

<213> Artificial Sequence

<220>

<223>

<400> 36

TCACCCTCGG AAGAAGCTGA TGAGA 25

<210> 37

<211> 25

<212> DNA

<213> Artificial Sequence

<220>

<223>

<400> 37

ATGGGACCTG TGCGGTTGGG AATAT  25

<210> 38

<211> 25

<212> DNA

<213> Artificial Sequence

<220>

<223>

<400> 38

TCAAAGATCT TCTCGGTCAA GTTTG  25

<210> 39

<211> 25

<212> DNA

<213> Artificial Sequence

<220>

<223>

<400> 39

ATGTTTTGCC CACTGAAACT CATCC  25

<210> 40

<211> 25

<212> DNA

<213> Artificial Sequence

<220>

<223>

<400> 40

TCATGAAAAT ATCCATTCTA CCTTG  25

<210> 41

<211> 25

<212> DNA

<213> Artificial Sequence

<220>

<223>

<400> 41

ATGGAACTGC TTCAAGTGAC CATTC  25

<210> 42

<211> 25

<212> DNA

<213> Artificial Sequence

<220>

<223>

<400> 42

TCAGTTCTTG GTTTTTCCTT GTGCA  25

<210> 43

<211> 25

<212> DNA

<213> Artificial Sequence

<220>

<223>

<400> 43

ATGCGACCCC AGGGCCCCGC CGCCT  25

<210> 44

<211> 25

<212> DNA

<213> Artificial Sequence

<220>

<223>

<400> 44

TTATTTTGGT AGTTCTTCAA TAATG 25
<210> 45
<211> 25
<212> DNA
<213> Artificial Sequence
<220>
<223>
<400> 45
ATGAAGTTAC AGTGTGTTTC CCTTT 25
<210> 46
<211> 25
<212> DNA
<213> Artificial Sequence
<220>
<223>
<400> 46
TCAGGAGGCC GATGGGGGCC AGCAC 25
<210> 47
<211> 25
<212> DNA
<220>
<223>
<213> Artificial Sequence
<400> 47
ATGGCCAGCC TGGGGCTGCT GCTCC 25
<210> 48
<211> 25
<212> DNA
<220>

<223>

<213> Artificial Sequence

<400> 48

TCATGAGGCT CCTGCAGAGG TCTGA  25

<210> 49

<211> 25

<212> DNA

<220>

<223>

<213> Artificial Sequence

<400> 49

ATGAAACTCC TGCTGCTGGC TCTTC  25

<210> 50

<211> 25

<212> DNA

<220>

<223>

<213> Artificial Sequence

<400> 50

TCATGAGCTA TGGTGAACAT TTGGA  25

<210> 51

<211> 25

<212> DNA

<213> Artificial Sequence

<220>

<223>

<400> 51

ATGGGCGGCC TGCTGCTGGC TGCTT  25

<210> 52

<211> 25
<212> DNA
<213> Artificial Sequence
<220>
<223>
<400> 52
CTACTGTGAC AGGAAGCCCA GGCTC 25
<210> 53
<211> 25
<212> DNA
<213> Artificial Sequence
<220>
<223>
<400> 53
ATGGCCCGGC ATGGGTTACC GCTGC 25
<210> 54
<211> 25
<212> DNA
<213> Artificial Sequence
<220>
<223>
<400> 54
TTACAGCTCC CCTGGCGGCC GGCCT 25
<210> 55
<211> 25
<212> DNA
<220>
<223>
<213> Artificial Sequence

<400> 55

ATGTGCTGGC TGCGGGCATG GGGCC 25

<210> 56

<211> 25

<212> DNA

<213> Artificial Sequence

<220>

<223>

<400> 56

TTATCTATTC ATCATATATT TCTTA 25

<210> 57

<211> 25

<212> DNA

<213> Artificial Sequence

<220>

<223>

<400> 57

ATGGGGTTCC CGGCCGCGGC GCTGC 25

<210> 58

<211> 25

<212> DNA

<213> Artificial Sequence

<220>

<223>

<400> 58

CTACGCCGAG ACCGTGGGCC TGCGG 25

<210> 59

<211> 25

<212> DNA

<213> Artificial Sequence

<220>

<223>

<400> 59

ATGCGAGGTG GCAAATGCAA CATGC   25

<210> 60

<211> 25

<212> DNA

<213> Artificial Sequence

<220>

<223>

<400> 60

TCATAAACTT GTGTTGGGCT TTAGG   25

<210> 61

<211> 36

<212> DNA

<213> Artificial Sequence

<220>

<223>

<400> 61

TCGGAATTCG CCATGGCCAA GTACCTGGCC CAGATC   36

<210> 62

<211> 60

<212> DNA

<213> Artificial Sequence

<220>

<223>

<400> 62

ACGCTCGAGT TACTTGTCAT CGTCGTCCTT GTAGTCCGTA TGGGGCATCT GCCCTTTTTC   60

<210> 63

<211> 36

<212> DNA

<213> Artificial Sequence

<220>

<223>

<400> 63

TCGGAATTCG CCATGGCCAG CCTGGGGCTG CTGCTC 36

<210> 64

<211> 60

<212> DNA

<213> Artificial Sequence

<220>

<223>

<400> 64

ACGCTCGAGT TACTTGTCAT CGTCGTCCTT GTAGTCTGAG GCTCCTGCAG AGGTCTGAGA 60

<210> 65

<211> 36

<212> DNA

<213> Artificial Sequence

<220>

<223>

<400> 65

TCGGAATTCA CCATGAAACT CCTGCTGCTG GCTCTT 36

<210> 66

<211> 60

<212> DNA

<213> Artificial Sequence

<220>

<223>

<400> 66

ACGCTCGAGT TACTTGTCAT CGTCGTCCTT GTAGTCTGAG CTATGGTGAA CATTTGGAAG 60

<210> 67

<211> 44

<212> DNA

<213> Artificial Sequence

<220>

<223>

<400> 67

TAGACGAATT CCCACCATGG GACCTGTGCG GTTGGGAATA TTGC 44

<210> 68

<211> 57

<212> DNA

<213> Artificial Sequence

<220>

<223>

<400> 68

AGGCAAGTCG ACAAGATCTT CTCGGTCAAG TTTGGGGTGG CTTCCTGTCT TGGTCAT 57

<210> 69

<211> 51

<212> DNA

<213> Artificial Sequence

<220>

<223>

<400> 69

GTGTAGAATT CCCACCATGG GCGGCCTGCT GCTGGCTGCT TTTCTGGCTT T 51

<210> 70

<211> 44

<212> DNA

<213> Artificial Sequence

<220>

<223>

<400> 70

CTGGGCGTCG ACCTGTGACA GGAAGCCCAG GCTCCTGCTC CACT 44